# EUROPEAN PATENT SPECIFICATION

(11) **EP 1 349 501 B1**
(45) Date of publication and mention of the grant of the patent: **11.03.2009**
(21) Application number: 01270132.2
(22) Date of filing: 11.12.2001
(51) Int. Cl.: A61B 17/00

(54) **DEVICES FOR SEALING OPENINGS THROUGH TISSUE AND APPARATUS FOR DELIVERING THEM**
VORRICHTUNGEN ZUM ABDICHTEN EINER ÖFFNUNG DURCH GEWEBE UND GERÄT ZUM EINFÜHREN DIESER VORRICHTUNGEN
DISPOSITIFS PERMETTANT D'OBTURER DES OUVERTURES PRATIQUEES DANS UN TISSU ET APPAREIL PERMETTANT D'ACHEMINER LESDITS DISPOSITIFS

(30) Priority: 14.12.2000 US 738431; 25.05.2001 US 866548; 15.08.2001 US 931676
(43) Date of publication of application: 08.10.2003
(73) Proprietor: Ensure Medical, Inc., Sunnyvale, CA 94085 (US)
(72) Inventor: GINN, Richard, S., San Jose, CA 95136 (US); SALMON, Stephen, M., Napa, CA 94558 (US); JABBA, Ronald, J., Redwood City, CA 94062 (US)
(74) Representative: McLeish, Nicholas Alistair Maxwell
(86) International application number: PCT/US2001/048843
(87) International publication number: WO 2002/062234

(56) References cited:
- US-A- 5 032 125
- US-A- 5 061 274
- US-A- 5 425 757
- US-A- 5 571 181
- US-A- 5 645 565
- US-A- 6 016 806

## Description

### FIELD OF THE INVENTION

The present invention relates generally to systems and methods for sealing or closing passages through tissue, and more particularly to devices for sealing punctures or other openings communicating with body lumens, such as blood vessels, and to apparatus and methods for delivering such devices.

### BACKGROUND

Catheterization and interventional procedures, such as angioplasty or stenting, generally are performed by inserting a hollow needle through a patient's skin and muscle tissue into the vascular system. A guide wire may then be passed through the needle lumen into the patient's blood vessel accessed by the needle. The needle may be removed, and an introducer sheath may be advanced over the guide wire into the vessel, e.g., in conjunction with or subsequent to a dilator. A catheter or other device may then be advanced through a lumen of the introducer sheath and over the guide wire into a position for performing a medical procedure. Thus, the introducer sheath may facilitate introduction of various devices into the vessel, while minimizing trauma to the vessel wall and/or minimizing blood loss during a procedure.

Upon completion of the procedure, the devices and introducer sheath may be removed, leaving a puncture site in the vessel wall. External pressure may be applied to the puncture site until clotting and wound sealing occur. This procedure, however, may be time consuming and expensive, requiring as much as an hour of a physician's or nurse's time. It is also uncomfortable for the patient, and requires that the patient remain immobilized in the operating room, catheter lab, or holding area. In addition, a risk of hematoma exists from bleeding before hemostasis occurs.

Various apparatus have been suggested for percutaneously sealing a vascular puncture by occluding the puncture site. For example, U.S. Patent Nos. 5,192,302 and 5,222,974, issued to Kensey et al., describe the use of a biodegradable plug that may be delivered through an introducer sheath into a puncture site. When deployed, the plug may seal the vessel and provide hemostasis. Such devices, however, may be difficult to position properly with respect to the vessel, which may be particularly significant since it is generally undesirable to expose the plug material, e. g., collagen, within the bloodstream, where it may float downstream and risk causing an embolism.

Another technique has been suggested that involves percutaneously suturing the puncture site, such as that disclosed in U. S. Patent No. 5,304,184, issued to Hathaway et al. Percutaneous suturing devices, however, may require significant skill by the user, and may be mechanically complex and expensive to manufacture.

Staples and surgical clips have also been suggested for closing wounds or other openings in tissue. For example, U. S. Patent Nos. 5,007,921 and 5,026,390, issued to Brown, disclose staples that may be used to close a wound or incision. In one embodiment, an"S"shaped staple is disclosed that includes barbs that may be engaged into tissue on either side of the wound. In another embodiment, a ring-shaped staple is disclosed that includes barbs that project from the ring. These staples, however, have a large cross-sectional profile and therefore may not be easy to deliver through a percutaneous site to close an opening in a vessel wall.

In addition, skin seals have been proposed that may be threaded into an opening in skin. For example, U. S. Patent No. 5,645,565, issued to Rudd et al., discloses a surgical plug that may be screwed into a puncture to seal the puncture. The surgical plug includes an enlarged cap and a threaded shaft that extends from the cap. During an endoscopic procedure, the plug may be threaded into an opening through skin until the cap engages the surface of the skin. The plug is intended to seal the opening communicating with a body cavity to prevent insufflation fluid from leaking from the cavity. Such plugs, however, may only be used at the surface of the skin, and may not be introduced through tissue, for example, to seal an opening in the wall of a blood vessel or other subcutaneous region.

In a further example US 5,425,757 discloses a method in which a plurality of closure elements or plugs are inserted into arterial tributaries. The closure elements comprise a recess at one end for receiving the operative head of a tool, such as a screwdriver, to facilitate insertion of the closure elements into the blood vessels.

US 5,690,674 discloses an elastic, biodegradable plug which has a proximal retainer and a distal retainer coupled by a waist. The plug may be positioned to straddle a wound in a blood vessel wall of a patient and therefore close the wound to stop bleeding. To position the plug in the wound, an operator may insert the plug into a sleeve extending through the vessel wall into-the-vessel and move the plug through the sleeve with a positioning tool. In the sleeve, the distal and proximal retainers of the plug are compressed. As the distal retainer of the plug exits the distal end of the sleeve into the vessel, the distal retainer expands in the vessel such that the surface of the distal retainer faces an inner side of the vessel wall. The positioning tool and the sleeve are then removed to position the waist in the wound of the vessel wall and allow the proximal retainer to expand such that a surface of the proximal retainer faces an outer side of the vessel wall.

In yet further examples, US 5,830,171 and WO 99/65544 disclose occluders in which a plug is inserted into the lacrimal duct in order to prevent the drainage of lacrimal liquid from the eyes. The former, US 5,830,171, discloses a punctal occluder for blocking the flow of lacrimal fluid from the surface of an eye through a lacrimal punctum. The punctual occluder includes a shank having a distal end for insertion into the lacrimal punctuin, and a distal flange attached to the distal end of the shank for insertion into the lacrimal punctum. The latter, WO 99/65544, discloses a canalicular plug including a through bore provided with a valve. The plug has a proximal occluding head, a central shaft and a distal body portion. The through bore is stepped and includes a large proximal portion for receiving an insertion tool, and a smaller distal portion. The valve is integrally molded with the canalicular plug, and the plug is made from a resilient bio-compatible material.

Accordingly, devices for sealing punctures or other passages through tissue, e.g., an opening into a blood vessel, would be considered useful.

### SUMMARY OF THE INVENTION

according to the present invention there is provided an apparatus for sealing a passage through a tissue as claimed in the appended claims.

The present invention is directed to an apparatus for sealing or closing passages through tissue, such as punctures communicating with blood vessels or other body lumens.

In accordance with one aspect of the present invention, an apparatus for sealing a passage through tissue is provided that includes an elongate member having a proximal end and a distal end, and a generally cylindrical plug member disposed on the distal end of the elongate member, the plug member including an outer surface which may have a helical thread thereon.

A hemostasis-promoting material, such as intestinal submucosa, or an infection-resistant material, may be secured to the plug member. The material may be delivered into the passage to facilitate hemostasis and/or heaving of tissue proximate the passage. A lumen is provided that extends from the proximal end of the elongate member through the plug member, and a seal may be provided for selectively sealing the lumen. The lumen may facilitate insertion of the apparatus and/or may facilitate monitoring hemostasis within the tissue.

The plug member may be releasable from the elongate member. Preferably, the plug member is formed from a bioabsorbable material such that the plug member may be released within the tissue to seal the passage, and allowed to be absorbed by the tissue over time. The elongate member preferably includes an actuator for releasing the plug member from the distal end of the elongate member. Preferably, cooperating connectors are provided on the distal end of the elongate member and the plug member for releasably securing the plug member to the distal end of the elongate member.

Alternatively, the elongate member may include an expandable member, such as a frame, on its distal end that may be selectively expanded to engage an interior wall of the plug member and collapsed to release the plug member.

The apparatus of the present invention may be used for sealing a passage through tissue. The apparatus provides an elongate member and a generally cylindrical plug member disposed on a distal end of the elongate member, the plug member including an outer surface may have a helical thread thereon.

The plug member is inserted into the passage, and the elongate member is rotated, thereby threading the plug member within the passage, the plug member engaging the tissue to substantially seal the passage. In one preferred method, the passage communicates with a blood vessel within the tissue, and the elongate member is rotated until the plug member substantially seals a wall of the blood vessel. The plug member may be left within the passage for sufficient time for hemostasis to occur, whereupon the plug member may be removed from the passage. In addition or alternatively, a hemostasis-promoting material may be provided within a cavity in a distal end of the plug member, and the material may be left in the passage when the plug member is removed. In a further alternative, the plug member itself may be released from the elongate member within the passage. Preferably the plug member is formed from a bioabsorbable material.

The elongate member may be rotated until it engages a blood vessel within the tissue. Blood flow at a location downstream of the blood vessel may be monitored, e. g., by feeling the patient's pulse. The elongate member may be rotated in a first direction to thread the plug member deeper into the passage until blood flow substantially ceases downstream of the blood vessel. Rotation of the elongate member may then be reversed to back the plug member a predetermined distance, thereby allowing blood flow to resume downstream of the blood vessel. The plug member may be released at this location or otherwise maintained at this location until hemostasis and/or healing occurs.

The apparatus may include an elongate guide member, a carrier member, and a plug member. The guide member may include a groove in an outer wall of the guide member that extends between proximal and distal portions of the guide member. The carrier member includes proximal and distal ends, and a first lumen extending between the proximal and distal ends.

The plug member, e. g., formed from bioabsorbable material, may be carried on the distal end of the carrier member. The plug member may be releasable from the distal end of the carrier member, e. g., by cooperating connectors,and/or an actuator, or may be substantially permanently attached to the carrier member. The plug member includes a second lumen extending therethrough that communicates with the first lumen, and may include a sealing member disposed in the second lumen for substantially sealing the second lumen from fluid flow therethrough. The plug member may include a substantially smooth outer surface, or may include a helical thread on its outer surface. Preferably, the plug member has a cross-section larger than a cross-section of the carrier member, e. g., to minimize dilation of a passage through which the carrier member is inserted.

The guide member is slidable into the first and second lumens such that edges of the groove substantially engage walls of the first and second lumens to define a bleed back lumen.

The apparatus of the present invention may be used in a method for sealing a passage through tissue communicating with a body lumen, e. g., a puncture communicating with a blood vessel. The passage may be used to access the body lumen, e. g., to perform an endovascular procedure within the patient's body. Upon completion of the procedure, any instruments received through the passage may be removed, although an introducer sheath and/or guidewire may remain in place through the passage into the vessel.

An elongate member including a groove extending between its proximal and distal portions may be inserted into the passage, e. g., through the introducer sheath or over the guidewire, until the distal portion enters the body lumen. The introducer sheath may be removed (if used), and a plug member disposed on a carrier member may be advanced over the elongate member with the elongate member received in a lumen of the plug member such that the groove and the lumen together define a bleed back lumen. The plug member may include an external thread, and consequently the carrier member may be rotated to thread the plug member into the passage.

When the plug member enters the body lumen, fluid from the body lumen may enter the bleed back lumen to identify the location of the body lumen with respect to the plug member. A first fluid flow rate through the groove may be detected when the distal portion of the elongate member initially enters the body lumen (before insertion of the plug member and carrier member), and a second greater fluid flow rate through the bleed back lumen may be detected when the plug member begins to enter the body lumen.

The plug member may be released from the carrier member within the passage, and the carrier member and/or guide member may be removed from the plug member. The plug member may include a sealing member for sealing the lumen upon removal of the guide member. Preferably, the plug member is formed at least partially from bioabsorbable material, and is left within the passage until it is absorbed by the tissue.

The plug member may include a tapered portion terminating in the distal end, and a helical thread pattern on an outer surface of the plug member. The plug member includes a lumen extending from the proximal end to a location proximal to the distal end. The plug member may be continuously tapered from the proximal end to the distal end, preferably having a frustoconical shape, defining a substantially blunt distal end. The lumen communicates with a distal port disposed on the distal end.

The apparatus may also include a carrier member or handle device including proximal and distal ends, the plug member being coupled to the distal end of the carrier member. The carrier member may include a lumen extending between the proximal and distal ends that communicates with the lumen in the plug member. The plug member may be formed from bioabsorbable material, and/or may be releasable from the distal end of the carrier member, similar to the embodiment described above.

A sealing member may be disposed within the lumen that is expandable for substantially sealing the lumen from fluid flow therethrough.

The sealing member may include a material that is expandable when exposed to fluid to substantially seal the lumen, such as a foam and/or a bioabsorbable material. The sealing member may be a valve or other device that is biased towards a first configuration for substantially sealing the lumen from fluid flow therethrough, and is movable to a second configuration for accommodating introduction of one or more devices through the lumen.

The lumen may include a tapered portion that tapers in crosssection, and the sealing member is a generally annular-shaped member disposed adjacent a wide end of the tapered portion of the lumen. The sealing member is movable into the tapered portion for substantially sealing the lumen from fluid flow therethrough.

The apparatus may include a handle device or other elongate member and a plug member. The elongate member has a proximal end, a distal end, and a lumen extending between the proximal and distal ends. The plug member is disposed on the distal end of the elongate member, and may include a helical thread on its outer surface and a distal port therein in communication with the lumen.

The plug member includes a passage therein extending between the distal port and the lumen. A sealing member may be disposed in the passage for substantially sealing the passage from fluid flow therethrough, such as that described above. In one embodiment, the passage and lumen define a bleed back lumen for determining the location of the plug member relative to a blood vessel or other body lumen.

In another embodiment, an obturator or other elongate member is insertable through the lumen such that a distal end of the obturator is disposed beyond the distal end of the plug member. The obturator may include a location indicator for identifying when the distal end of the plug member is disposed adjacent a body lumen. The location indicator may include a bleed back lumen in the obturator and a bleed back port on its distal tip, the bleed back port being in communication with the bleed back lumen. Alternatively, the location identifier may include an expandable member on a distal tip of the obturator, the expandable member being expandable when the distal tip is disposed within a body lumen for providing tactile feedback of a location of the distal end of the plug member with respect to the body lumen.

Preferably, the plug member is releasable from the elongate member. The elongate member may include an actuator for releasing the plug member from the distal end of the elongate member. Preferably, cooperating connectors are provided on the distal end of the elongate member and on the plug member for releasably securing the plug member to the distal end of the elongate member.

In use the plug member may be inserted into the passage until the helical thread begins to enter the passage. The elongate member may be rotated in a first direction, thereby threading the plug member into the passage until the bleed back indicator enters the body lumen, whereupon fluid from the body lumen may enter the bleed back indicator to identify the location of the body lumen with respect to the plug member. If desired, rotation of the elongate member may be reversed, thereby withdrawing the plug member a predetermined distance relative to the body lumen. Thereafter, the plug member may be released from the elongate member within the passage. Preferably, the plug member is formed from bioabsorbable material, and the plug member is left within the passage until it is absorbed by the tissue.

In a preferred embodiment, the elongate member includes a lumen extending from its proximal end through the plug member, and the bleed back indicator includes a bleed back port in the plug member, the bleed back port being in communication with the lumen. Alternatively, an obturator may be inserted through the lumen until a distal end of the obturator extends distally beyond the plug member, and the bleed back indicator may include a bleed back lumen in the obturator.

A sealing member may be provided in a lumen of the plug member for sealing the lumen, and consequently the bleed back port, from fluid flow therethrough. The sealing member may be an expandable material that expands when exposed to fluid. Alternatively, the sealing member may be a generally annular-shaped element that may be disposed adjacent a wide end of a tapered portion of the lumen. The annular-shaped may be moved or otherwise wedged into the tapered portion for substantially sealing the lumen.

Other objects and features of the present invention will become apparent from consideration of the following description taken in conjunction with the accompanying drawings.

### BRIEF DESCRIPTION OF THE DRAWINGS

FIG. 1 is a perspective view of an apparatus for sealing a passage through tissue.
FIG. 2 is a cross-sectional side view showing a plug member on the apparatus of FIG. 1 being used to deliver a patch to seal an opening in a wall of a blood vessel.
FIG. 3 is a perspective view of an embodiment of an apparatus for sealing a passage through tissue, in accordance with the present invention.
FIG. 4 is a perspective view of an alternative arrangement, including a detachable plug, in accordance with the present invention.
FIGS.5A-5C are cross-sectional views of the apparatus of FIG. 4 being used to deliver the plug into a passage communicating with a blood vessel.
FIGS. 6A and 6B are cross-sectional views of another detachable plug and a delivery apparatus including an expandable frame shown in expanded and collapsed states, respectively, for securing and releasing the plug member.
FIG. 7A is an exploded perspective view of another preferred embodiment of an apparatus for sealing a passage through tissue, in accordance with the present invention.
FIG. 7B is a perspective view of the assembled apparatus of FIG. 7A.
FIG. 8A is a detail of a plug member on a distal end of the apparatus of FIGS. 7A and 7B.
FIG. 8B is a detail of an alternative embodiment of a plug member on a distal end of the apparatus of FIGS. 7A and 7B.
FIG. 9A is a cross-sectional view of the apparatus of FIG. 7B taken along line 9A9A.
FIGS. 9B-9F are cross-sectional views of the apparatus of FIG. 7B, showing alternate embodiments of a guide member.

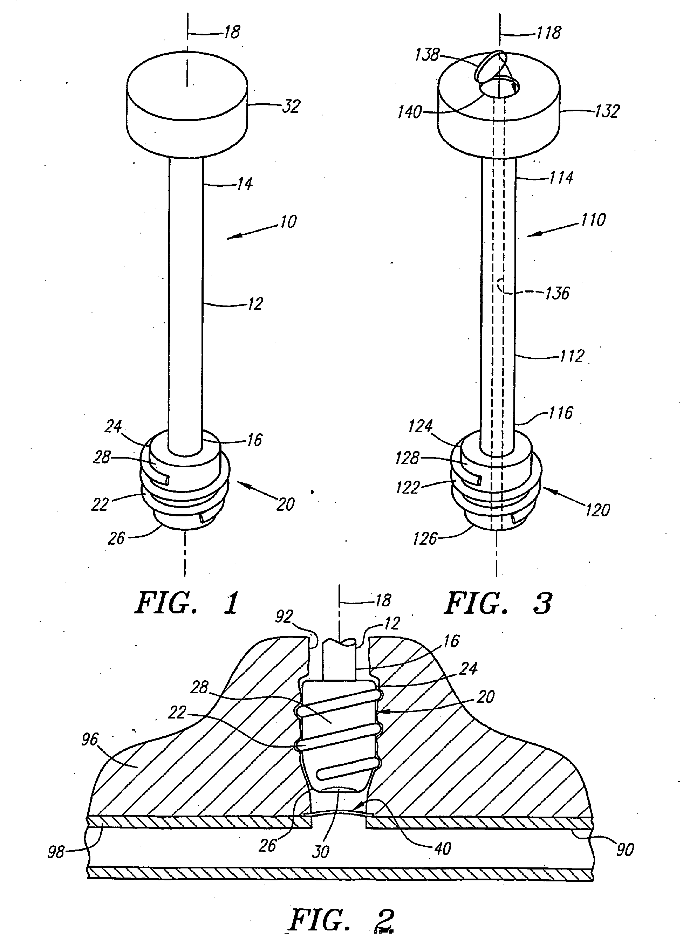

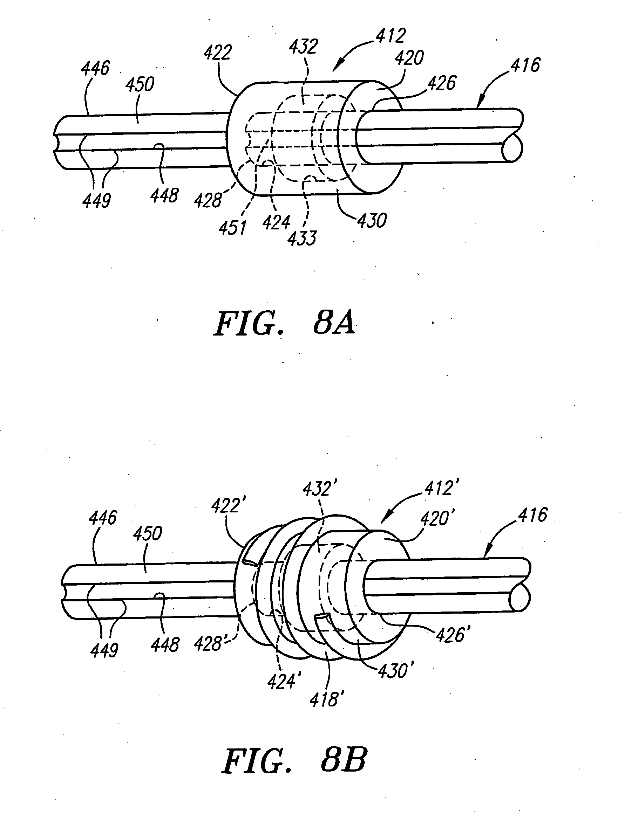

FIGS.1 OA-1 OD are cross-sectional views showing a plug member on the apparatus of FIGS. 8A and 8B being used to seal an opening in a wall of a blood vessel.

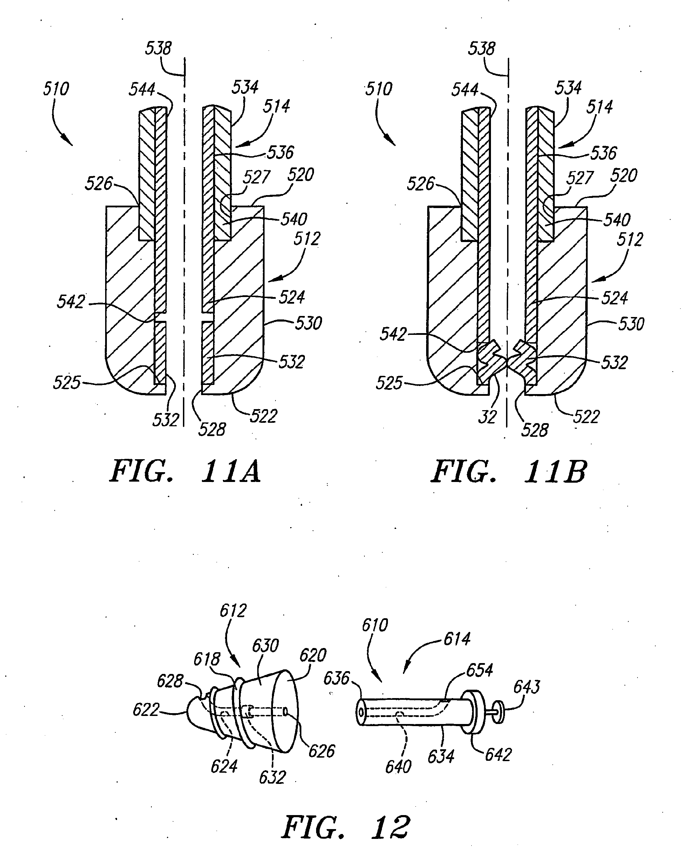

FIGS.11A and 11B are cross-sectional views of a distal end of a third preferred embodiment of a plug member, in accordance with the present invention.
FIGS. 12 is an exploded perspective view of yet a further embodiment of an apparatus for sealing a passage through tissue, in accordance with the present invention.

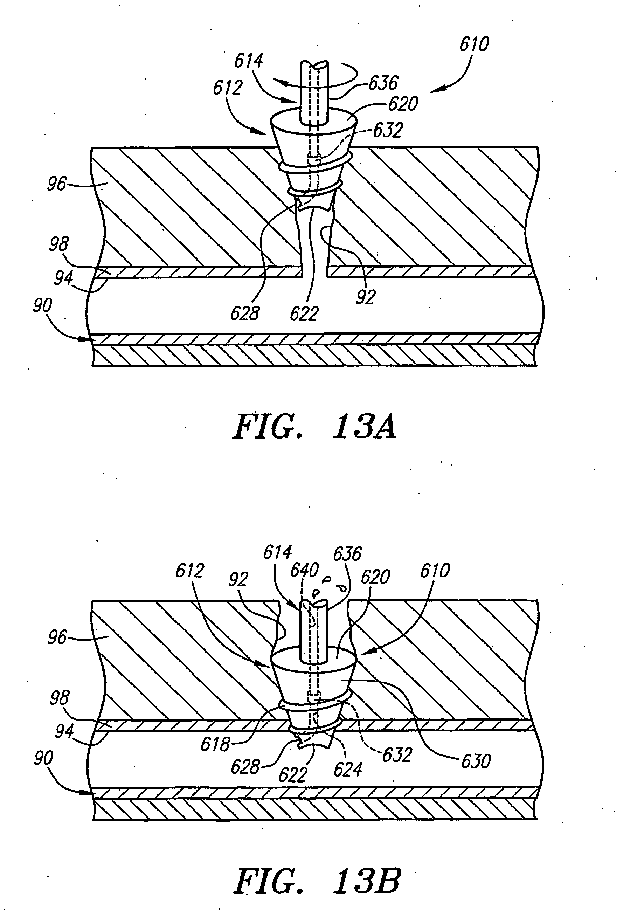

FIGS. 13A and 13B are cross-sectional views showing a plug member on the apparatus of FIG. 12 being used to seal an opening in a wall of a blood vessel.

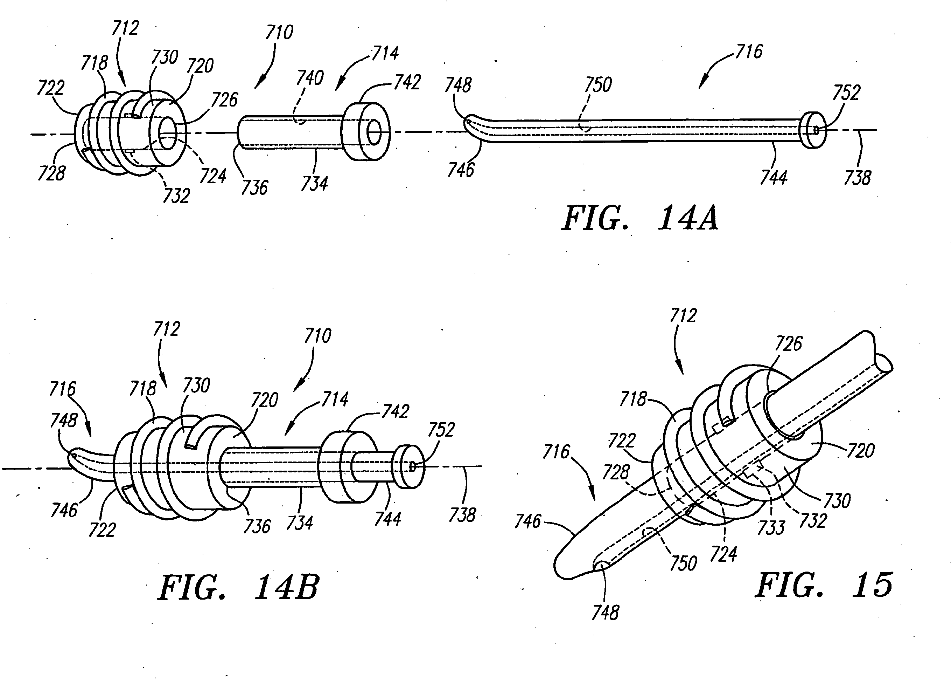

FIG. 14A is an exploded perspective view of a further embodiment of an apparatus for sealing a passage through tissue, in accordance with the present invention.
FIG. 14B is a perspective view of the assembled apparatus of FIG. 14A.
FIG. 15 is a detail of a distal end of the apparatus of FIGS. 14A and 14B.

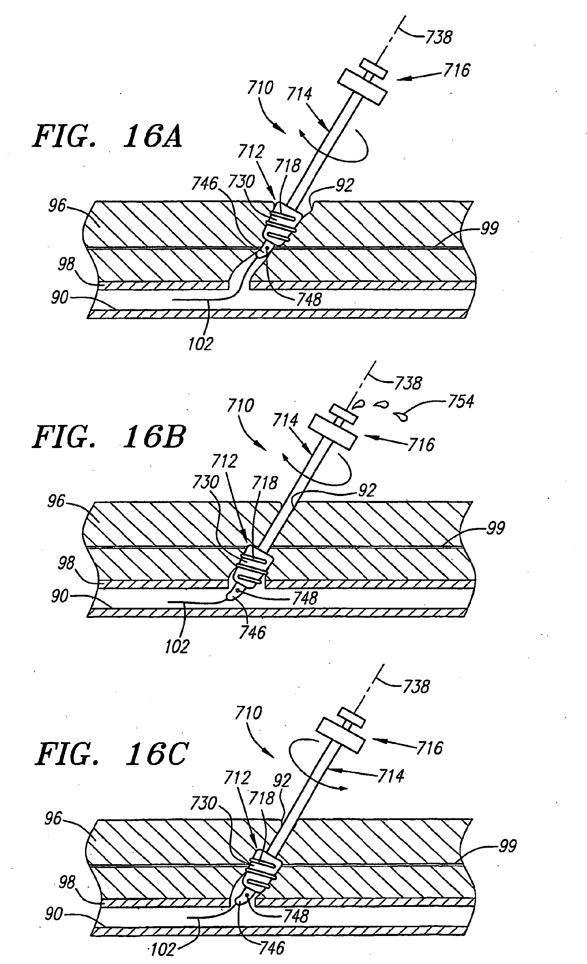

FIGS.16A-16D are cross-sectional views showing a plug member on the apparatus of FIGS. 14A and 14B being used to seal an opening in a wall of a blood vessel.

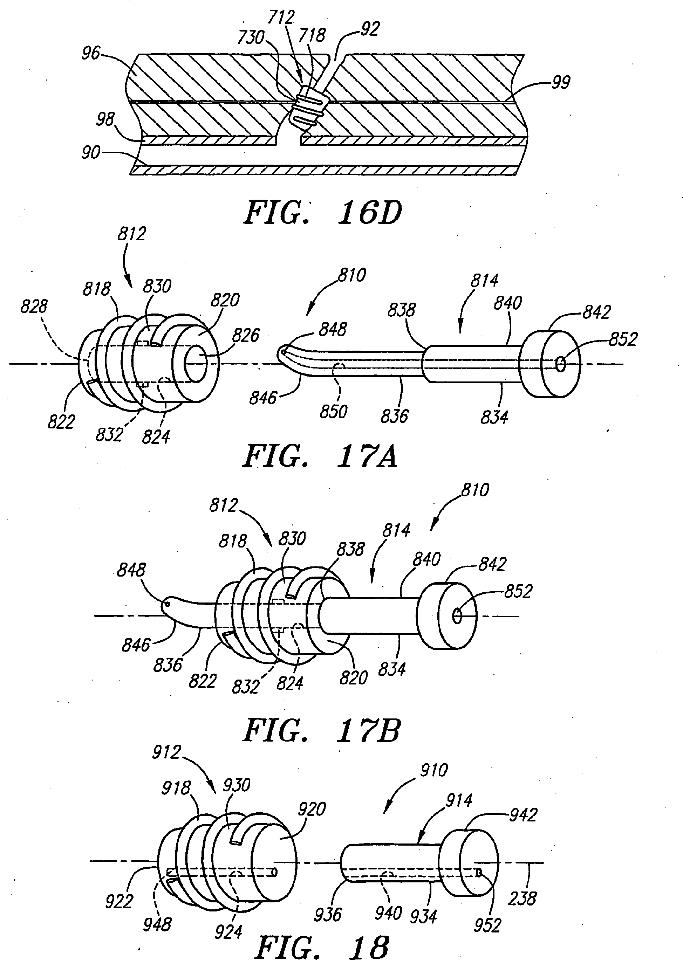

FIGS. 17A is an exploded perspective view of yet another embodiment of an apparatus for sealing a passage through tissue, in accordance with the present invention.
FIG. 17B is a perspective view of the assembled apparatus of FIG. 17A.
FIG. 18 is a side view of yet another embodiment of an apparatus for sealing a passage through tissue, in accordance with the present invention.

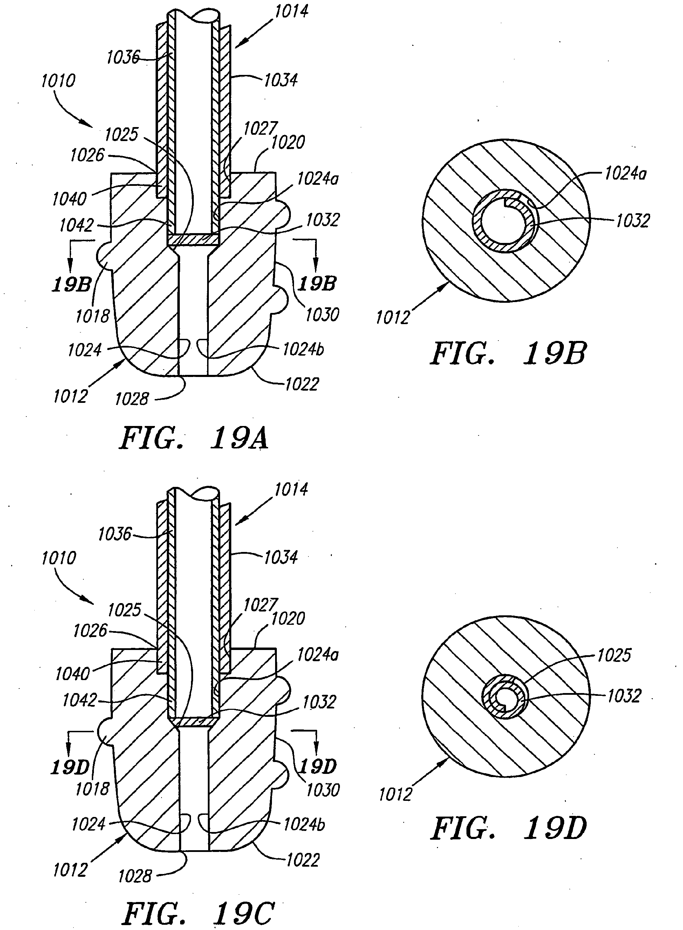

FIG. 19A is a cross-sectional side view of yet a further embodiment of an apparatus for sealing a passage through tissue, including a plug member with a sealing member within its lumen, in accordance with the present invention.
FIG. 19B is a cross-sectional detail of the plug member of FIG. 19A, taken along line19B-19B, showing the sealing member in an open configuration.
FIG. 19C is a cross-sectional side view of the apparatus of FIG.19A, with the sealing member wedged into, a tapered portion of the lumen.
FIG. 19D is a cross-sectional detail of the plug member of FIG. 19C, taken along line 19D-19D, showing the sealing member wedged into a closed configuration.

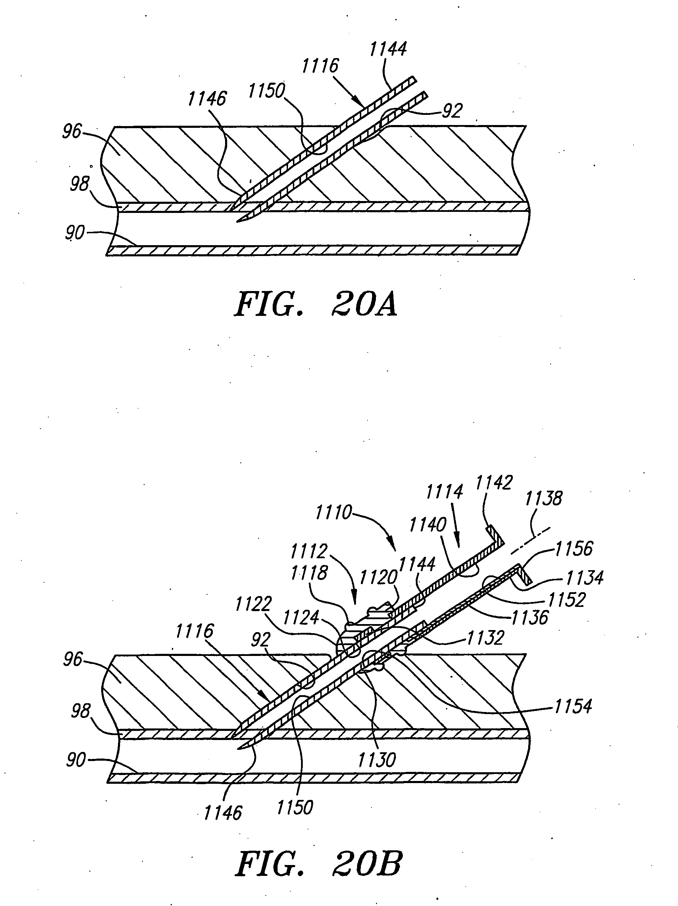

FIGS. 20A-20F are cross-sectional views showing an apparatus and method four delivering a plug member to seal a puncture communicating with a blood vessel.

### DETAILED DESCRIPTION OF THE PREFERRED EMBODIMENTS

Turning now to the drawings, FIG. 1 shows an apparatus 10 for sealing a passage through tissue. Generally, the apparatus 10 includes an elongate shaft 12 and a plug member 20. The shaft 12 has a proximal end 14 and a distal end 16 defining a longitudinal axis 18 therebetween. A handle 32 may be provided on the proximal end 14 of the shaft12 for facilitating manipulation of the apparatus 10, e. g., to facilitate rotation of the apparatus 10 into a passage, as described below.

The plug member 20 is disposed on the distal end 16 of the shaft 12 and includes a helical thread 22 extending at least partially between its proximal and distal ends 24,26. The plug member 20 is a substantially rigid body having a generally cylindrical shape and defining an outer surface 28 along which the helical thread 22 extends. The helical thread 22 preferably is substantially rigid and has a substantially square cross-section to facilitate sealing of a passage into which the plug member 20 is threaded. Alternatively, the plug member 20 may be a semi-rigid or flexible body or may have a substantially flexible distal tip (not shown), e. g., to facilitate atraumatic insertion of the plug member 20 into the passage.

Preferably, the shaft 12 has a cross-section that is substantially smaller than a cross-section of the plug member 20, e. g., to minimize dilation of a passage into which the apparatus 10 is inserted. The plug member 20 may be tapered between the proximal and distal ends 24,26and/or the distal end 26, or only partially tapered, e. g., at the distal end 26. In addition or alternatively, the distal end 26 may be rounded to facilitate advancement of the plug member 20 into a passage through tissue.

In one arrangement, the helical thread 22 is integrally formed on the outer surface 28 of the plug member 20. For example, the plug member 20 and thread 22 may be formed by injection molding. Alternatively, the threads may be cut or otherwise formed in the outer surface 28 of the plug member 20. The plug member 20 may be formed from a biocompatible material, preferably a plastic, such as polyethylene or polyester. The plug member 20 may be substantially permanently attached to the distal end 16 of the shaft 12, for example, by mechanical detents and/or an adhesive. Alternatively, the shaft 12 and plug - member 20 may also be integrally formed as a single piece. In a further alternative, the plug member 20 may releasably attached to the shaft 12, as described below.

In one arrangement, the plug member 20 includes a cavity, such as concave recess 30, in its distal end 26. A material (not shown) may be provided in the cavity 30, such as intestinal submucosa, collagen, an infection-resistant material, and the like. Such material may promote hemostasis and/or healing of the tissue, as will be appreciated by those skilled in the art. Alternatively, the material may be otherwise detachably secured to the distal end 26 of the plug member 20, either within the cavity 30 or across the distal end 26 without a cavity (not shown). For example, the material may be secured using a biodegradable adhesive or a mechanical fastener, such as one or more clips (not shown). Such a fastener may be actuated from the proximal end 14 of the shaft 12 to release the material, or the material may otherwise be automatically released upon withdrawal of the plug member 20.

Turning to FIG. 2, during use, the plug member 20 may be used to seal and/or close a passage through tissue 96, such as a puncture 92 communicating with a blood vessel 90 or other body lumen. The puncture 92 may be used to provide percutaneous access to the blood vessel 90. For example, the puncture 92 may facilitate performing an endovascular procedure within a patient's vasculature, such as angioplasty, stenting, atherectomy, and the like, or may otherwise provide access via the blood vessel 90 to a region within the patient's body. Upon completion of the procedure, any instruments, such as an introducer sheath (not shown), may be removed from the blood vessel 90 and puncture 92.

The apparatus 10 may then be introduced into the puncture 92, for example, by at least partially inserting the plug member 20 into the puncture 92. Preferably, the outer surface 28 and threads 22 engage tissue 96 surrounding the puncture 92, thereby substantially sealing the puncture 92 from fluids, such as blood, within the vessel 90. The apparatus 10 may then be rotated in a first direction about its longitudinal axis 18 to thread the plug member 20 substantially atraumatically deeper into the puncture 92. Preferably, the plug member 20 is advanced until its distal end 26 is proximate to the wall 98 of the vessel 90.

In the arrangement shown in FIG. 2, a patch 40, e. g., of intestinal submucosa or other hemostasis-promoting material, is provided in a cavity 30 within the distal end 26 of the plug member 20. For example, U. S. Patent No. 2,167,251, issued to Rogers, discloses exemplary materials that may be included in the cavity 30 or otherwise detachably secured to the plug member 20.

When the plug member 20 is advanced proximate the wall 98 of the vessel 90, the patch 40 is delivered within or adjacent to the wall 98. The patch 40 may promote hemostasis and/or healing of the wall 98 at the puncture site. After hemostasisand/or a desired level of healing has occurred, the plug member 20 may be withdrawn from the puncture 92 by rotating the apparatus 10 in a second direction opposite the first direction. Alternatively, the patch 40 may be delivered to the wall 98 at the puncture site simply by releasably securing the patch 40 to the distal end 26 of the plug member 20, as described above. Alternatively, the plug member 20 may be advanced into the puncture 92 without a patch to seal the puncture and allow hemostasis and/or healing to occur naturally before removal of the plug member 20.

Turning to FIG. 3, according to an embodiment of the present invention there is shown an apparatus 110 for sealing a passage through tissue. Similar to the previous arrangement, the apparatus 110 includes an elongate shaft 112, a plug member 120 disposed on a distal end 116 of the shaft 112, and a handle 132 on a proximal end 114 of the shaft 112. In addition, the apparatus 110 includes a lumen136 extending from the proximal end 114 of the shaft 112 distally through the plug member 120. A seal 138, such as a hinged cap, may be provided for sealing an opening 140 communicating with the lumen 138. The seal 138 and opening 140 may be provided on the handle 132 or elsewhere on the proximal end 114 of the shaft 112.

Use of the apparatus 110 is similar to the previous arrangement, i. e., the plug member-120 is threaded into a puncture, e. g., to seal an opening in a wall of a blood vessel (not shown). The seal 138 is initially provided closed, thereby preventing blood or other fluid from passing through the lumen 136 to the proximal end 114 of the shaft 112. Alternatively, the cap 138 may be initially provided open, such that when the distal end 126 of the plug member 120 starts to enter a blood vessel, blood within the vessel may enter and pass proximally through the lumen and exit the opening 140, thereby providing a visual indicator that the plug member 120 has reached the vessel. The seal 138 may then be sealed to prevent further flow of fluid therethrough.

Periodically, the seal 138 may be opened to see if fluid flows from within the vessel through the lumen 136. Once hemostasis occurs, such flow may no longer occur when the seal138 is opened, thereby indicating that the apparatus 110 may be removed. Alternatively, the lumen 136 may be coupled to another tube or other external system that may facilitate monitoring of fluid flow through the lumen 136.

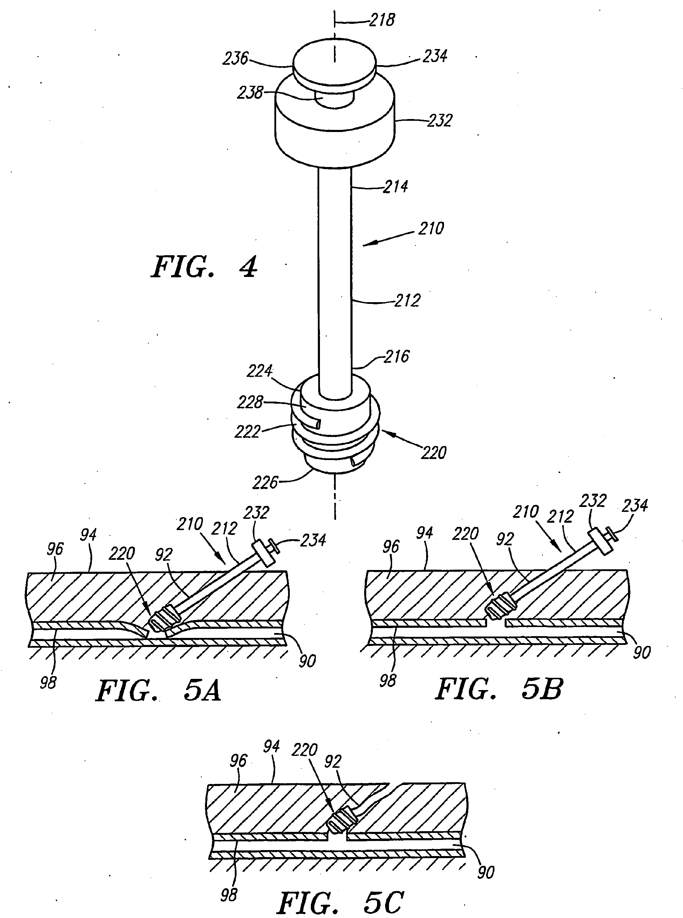

Turning to FIG. 4, another arrangement of an apparatus 210 is shown for sealing a passage through tissue. Similar to the previous embodiment, the apparatus 210 includes an elongate shaft 212, a plug member 220 disposed on a distal end 216 of the shaft 212, and a handle 232 on a proximal end 214 of the shaft 212. Unlike the previous embodiment, however, the plug member 220 is releasable from the shaft 212. The plug member 220 preferably is at least partially formed from bioabsorbable material, such as collagen,PGA's, PLA's, and the like, that may be at least partially absorbed by the patient's body over time.

The plug member 220 and the distal end 216 of the shaft 212 generally include cooperating connectors (not shown) for releasably securing the plug member 220 to the shaft 212. For example, the plug member 220 may include a recess (not shown) in its proximal end 224 and the shaft 212 may include a mechanism, e. g., radially projecting fingers, for frictionally engaging the wall of the recess. Alternatively, the recess may include slots for positively receiving the mechanism on the shaft 212. In a further alternative, the plug member 220 may include a hub (not shown) extending from its proximal end 224 and the shaft 212 may include a mechanism for detachable securing the hub to the shaft 212.

Preferably, the handle 232 includes an actuator 234 that may be activated to release the connectors securing the plug member 220 to the shaft 212. For example, the actuator 234 may include a button236 coupled to a control rod or wire238 that extends through then shaft 212 to its distal end 216. Upon depression of the button 236, the control rod 238 may be moved, thereby disengaging the connector on the shaft 212 from the mating connector on the plug member 220. In another alternative, the distal end 216 of the shaft 212 and the plug member 220 may include mating threads (not shown) so that the shaft 212 may be rotated with respect to the plug member 220 to release the plug member 220. In this arrangement, the mating threads should wind helically in the same direction as the helical threads 224 to ensure that the plug member 220 is not released prematurely from the shaft 212.

Turning to FIGS.5A-5C, the apparatus210 may be used to seal a passage 92 extending from a patient's skin 94 through tissue 96 to a blood vessel 90 or other body lumen. The passage 92 may be formed, a procedure performed within the patient's body via the vessel 90, and any instruments removed from the passage 92. The plug member 220 may be introduced into the passage 92, and the apparatus 210 may be rotated to thread the plug member 220 into the passage 92. Preferably, the threads 222 facilitate controlled advancement of the plug member 220 through the tissue 94 and/or facilitate substantially sealing of fluid flow through the passage 92.

Preferably, while advancing the plug member 220, blood flow at a location downstream of the blood vessel 90 is monitored. For example, the patient's pulse may be manually or automatically monitored downstream of the puncture site. The apparatus 210 may be rotated, advancing the plug member 220 until blood flow substantially ceases downstream of the blood vessel. This may indicate that the plug member 220 has engaged and compressed the blood vessel 90, as shown in FIG. 5A.

Rotation of the apparatus 210 may then be reversed to back the plug member 220 a predetermined distance, thereby allowing blood flow to resume downstream of the blood vessel 90. For example, the threads 222 may have a predetermined thread spacing such that the apparatus 210 may be rotated a predetermined number of times to accurately withdraw the plug member 220 from compressing the blood vessel 90 while still substantially sealing the passage 92 at the wall 98 of the vessel 90, as shown in FIG. 5B.

The plug member 220 may then be released from the shaft 212, e. g., by depressing the actuator 234, and the shaft 212 withdrawn from the passage 92, leaving the plug member 220 within the passage 92, as shown in FIG. 5C. Because the plug member 220 is bioabsorbable, the plug member 220 may be absorbed by the tissue over time, thereby allowing the wall 98 of the vessel 90 and tissue 96 surrounding the passage 92 to at least partially heal before the plug member 220 is absorbed.

In an alternative arrangement, the pug member 220 may be biocompatible, but not bioabsorbable. In this alternative, it may be desirable or necessary to subsequently remove the plug member 220, e.g., once hemostasis and/or healing of the wall98 of the vessel 90 has occurred. The plug member 220 may be retrieved by creating an incision in the patient's skin 94 and/or through the tissue 96 (e. g., if the passage has healed), and introducing an apparatus (not shown) to retrieve the plug member 220, such as a shaft having a connector on its end, similar to the apparatus210 used to deliver the plug member 220.

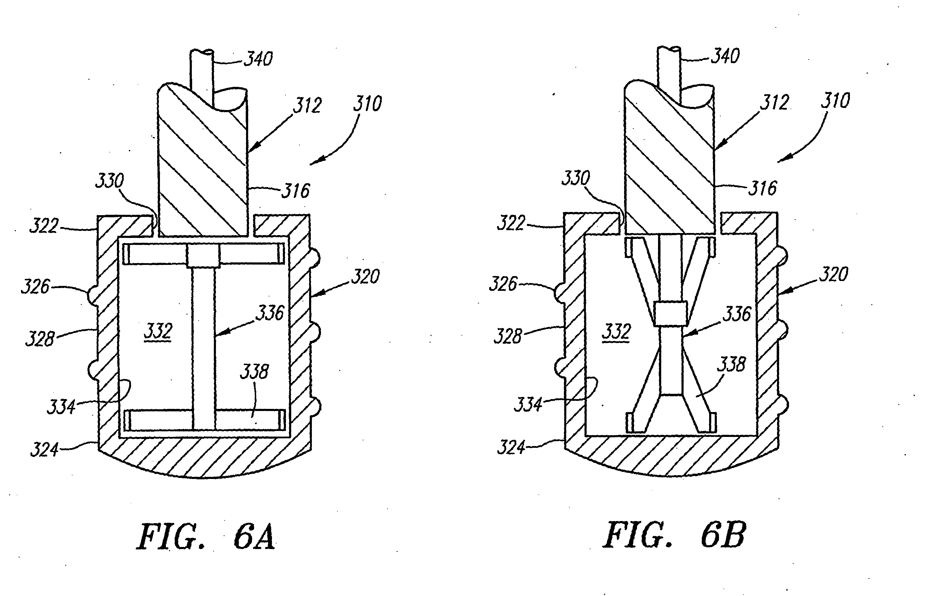

Turning to FIGS. 6A and 6B, yet another arrangement of an apparatus 310 is shown for sealing a passage through tissue. Similar to the previous embodiment, the apparatus 310 includes an elongate shaft 312, a plug member 320 disposed on a distal end 316 of the shaft 312, and a handle (not shown) on a proximal end (also not shown) of the shaft 312.

The plug member 320 is preferably at least partially formed from bioabsorbable material that may be absorbed by the patient's body over time, similar to the embodiment described previously. The plug member 320 includes proximal and distal ends 322,324 and a helical thread 326 extending along an exterior surface 328 thereof.hi addition, an opening 330 in the proximal end 322 communicates with an interior cavity 332 defined by an interior wall 334.

An expandable member 336 extends from the distal end 316 of the shaft 312 for securing the plug member 320 to the shaft 312 during delivery. In one arrangement, the expandable member 336 is a mechanically expandable frame including a plurality of radially movable arms 338. Preferably, the handle on the shaft 312 includes an actuator (not shown) that may be activated to control an actuator rod or wire 340, to selectively collapse and/or expand the frame 336 to release or secure the plug member 320 to the shaft 312, respectively.

The frame 336 may be collapsed and inserted through the opening 330 into the interior cavity 332 of the plug member 320. The frame 336 may then be expanded to an expanded state, shown in FIG. 6A, to engage the interior wall 334 of the plug member 320, thereby substantially securing the plug member 320 to the distal end 316 of the shaft 312. Subsequently, the frame 336 may be selectively collapsed to a collapsed state, shown in FIG. 6B, for releasing the plug member 320 from the shaft 312. In the collapsed state, the frame336 may provided a relatively low cross-section or profile, thereby facilitating removal of the shaft 312 from a passage within which the plug member 320 is delivered.

In the expanded state, the radial arms 338 of the frame 336 may engage the interior wall 334 of the plug member 320 at a plurality of discrete locations. In this form, the plug member 320 is preferably sufficiently rigid that it is self supporting when the frame 336 is collapsed. Alternatively, the radial arms 332 may support elongate bands (not shown), e. g., that extend between respective arms 322 that may provide greater surface contact with the interior wall 334 of the plug member 320. These bands may extend circumferentially and/or axially between arms 338 of the frame 336. Thus, the frame 336 may provide structural support for the plug member 320, e. g., during advancement of the apparatus310 into a passage through tissue (not shown).

In an alternative arrangement, the expandable member may be an inflatable member (not shown), such as an elastic or inelastic balloon. Preferably, the balloon is substantially inelastic such that, when the balloon is fully expanded, it has a shape similar to the shape of the interior cavity 332 within the plug member 320. The balloon may engage the interior wall 334 of the plug member 320 to substantially support the plug member 320 during delivery. The balloon may be selectively inflated or deflated to secure the plug member 320 on the shaft 312 or to release the plug member 320 from the shaft 312, respectively.

The plug member 320 may be delivered to seal a passage through tissue, for example, using the method described above. The expandable member 330 (whether mechanical or inflatable) may be expanded to secure the plug member 320 on the shaft 312. The plug member 320 may be introduced into a passage, and the apparatus 310 may be rotated to thread the plug member 320 into the passage. Once advanced to a desired location, the plug member 320 may be released from the shaft 312, e. g., by collapsing the expandable member 330. The shaft 312 may then be withdrawn from the passage, leaving the plug member 320 within the passage.

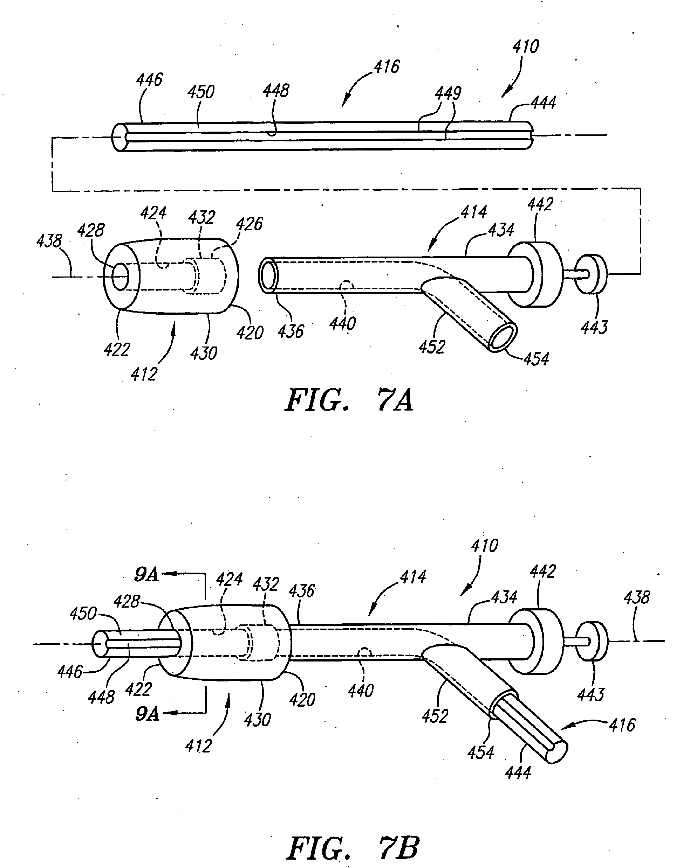

Turning to FIGS. 7A and 7B, another preferred embodiment of an apparatus 410 for sealing a passage through tissue, in accordance with the present invention. Generally, the apparatus 410 includes a plug member 412, an elongate shaft or carrier member 414, and an elongate guide member 416.

The plug member 412 is a substantially rigid body, preferably having a generally cylindrical shape, including a proximal end 420, a distal end 422, and an outer surface 430. The plug member 412 includes a lumen 424 that extends between a proximal opening 426 and a distal opening or port 428.

The plug member 412 may be formed from a biocompatible material, e.g., a plastic, such as polyethylene or polyester. Preferably, the plug member 412 is formed at least partially (and more preferably entirely) from bioabsorbable material, such as collagen, polyglycolic acids (PGA's), polyactides (PLA's), and the like, that may be at least partially absorbed by the patient's body over time. Alternatively, the plug member 412 may be a semi-rigid or flexible body or may have a substantially flexible distal tip (not shown), e. g., to facilitate atraumatic insertion of the plug member 412 into the passage. In addition or alternatively, the plug member 412 may be tapered along its length, and/or the distal end 422 may be rounded to facilitate advancement of the plug member 412 into a passage through tissue.

Preferably, the plug member 412 has a length of not more than about ten millimeters. (10 mm), and more preferably between about one and ten millimeters (1-10 mm). The plug member 412 also preferably has a diameter of between about one and twenty millimeters (1-20 mm). In addition, the length and diameter may have a ratio that is not more than about two-to-one.

In one embodiment, shown in FIG. 8A, the outer surface 430 is substantially smooth, thereby facilitating direct advancement of the plug member 412 into a passage through tissue (not shown). In an alternative embodiment, shown in FIG. 8B, the plug member 412'may include a helical thread pattern 418', including one or more helical threads, that extend at least partially between its proximal and distal ends 420', 422'. The thread pattern 418'may extend completely to the distal end 422'of the plug member412', and may be tapered at the distal end 422'to facilitate introduction into a passage through tissue (not shown). Additional information on plug members including an external helical thread pattern may be found in US Patent Nos 6,663,655 and 6,846,319.

Returning to FIG. 8A (in which the carrier member 414 has been eliminated for convenience), a sealing member 432 may be provided within the lumen 424 for substantially sealing the lumen 424 from fluid flow therethrough. In a preferred embodiment, the sealing member 432 has an annular shape, and is mounted within an annular recess 433 in the lumen 424. The sealing member 432 is preferably formed from a material that expands when exposed to fluids, e. g., an expandable foam. More preferably, the sealing member 432 is also bioabsorbable, similar to the plug member 412 itself. Exemplary materials that may be appropriate for use in the sealing member 432 and/or for the plug member 412 are disclosed in U. S. Patent No. 6,224,630, the disclosure of which is expressly incorporated herein by reference. Alternatively, the sealing member 432 may be a valve (not shown) that is biased to substantially seal the lumen 424 from fluid flow, but may be opened to facilitate introduction of one or more devices, e. g., the obturator 416 therethrough, as described further below.

In addition, the plug member 412 may include a cavity (not shown) in the distal end 422. A material (also not shown) may be provided in the cavity, such as extra-cellular matrix material, e. g., intestinal, stomach, or bladder submucosa, collagen, an infection-resistant material, and the like, that may promote hemostasis and/or healing of the tissue. Alternatively, such material may be otherwise detachably secured to the distal end 422 of the plug member 412, either within a cavity or across the distal end 422 without a cavity. For example, the material may be secured using a biodegradable adhesive or a mechanical fastener, such as one or more clips (not shown).

Returning to FIGS. 7A and 7B, the carrier member 414 has a proximal end 434 and a distal end 436, and defines a longitudinal axis 438 that extends between the proximal and distal ends 434,436. A lumen 440 also extends between the proximal and distal ends 434,436 for accommodating insertion of the guide member 416 therethrough, as described further below. A handle 442 may be provided on the proximal end 434 of the carrier member 414 for facilitating manipulation of the apparatus 410.

Preferably, the carrier member 414 is a substantially rigid tubular member, formed from a biocompatible material, e. g., plastic, such as polyethylene or polyester, or metal, such as stainless steel. The carrier member 414 preferably has a cross-section that is substantially smaller than a cross-section of the plug member 412, e. g., to minimize dilation of a passage into which the apparatus 410 is inserted. In the preferred embodiment shown, the carrier member 414 may include a lateral proximal portion 452 through which the lumen 440 extends and communicates with a proximal port 454. The proximal port 454 may be connected to a valve or other device (not shown) for facilitating visual observation of fluid exiting the proximal port 454, as will be appreciated by those skilled in the art.

At least one of the plug member 412 and the distal end 436 of the carrier member 414 includes a connector. Preferably, the plug member 412 and the distal end 436 of the carrier member 414 include cooperating connectors (not shown) for releasably securing the plug member 412 to the carrier member 414, as described in US Patent No. 6,846,319. Preferably, the cooperating connectors substantially couple the plug member 412 to the carrier member 414 such that the plug member 412 cannot move independently of the carrier member 414, e. g., such that the plug member 412 may be rotated only by rotating the carrier member 414.

Preferably, the handle 442 includes an actuator 443 that may be activated to release the connector (s) securing the plug member 412 to the distal end 436 of the carrier member 414. For example, the actuator 443 may include a button coupled to a control rod or wire (not shown) that extends through the carrier member 414 to the distal end 436. Upon depression of the button, the control rod may be moved, thereby disengaging the connector on the carrier member 414 from the plug member 412 or a mating connector (not shown) on the plug member 412. In another alternative, the distal end 436 of the carrier member 414 and the plug member 412 may include mating threads (not shown) such that the carrier member 414 may be rotated with respect to the plug member 412 to release the plug member 412. In this embodiment, the mating threads should wind helically in the same direction as the thread pattern 418 on the plug member 412 to ensure that the plug member 412 is not released prematurely from the carrier member 414.

The guide member 416 is an elongate member, e.g., formed from flexible or semi-rigid material, having a proximal end 444 and a substantially atraumatic and/or flexible distal tip 446, e.g., having a size for insertion into a passage through tissue into a blood vessel or other body lumen (not shown). An elongate groove 448 defined by opposing edges 449 is provided in an external surface 450 that extends from the proximal end 444 to the distal tip 446. Alternatively, the groove 448 may terminate before to the distal tip 446, e.g., a predetermined distance proximal to the distal tip 446 (not shown).

The guide member 416 has a size and shape for slidable insertion through the lumen 440 of the carrier member 414 and through the lumen 424 of the plug member 412. During use, the guide member 416 may be inserted through the carrier member 414 such that the distal tip 446 of the guide member 416 extends beyond the distal end 422 of the plug member 412, as shown in FIG. 7B.

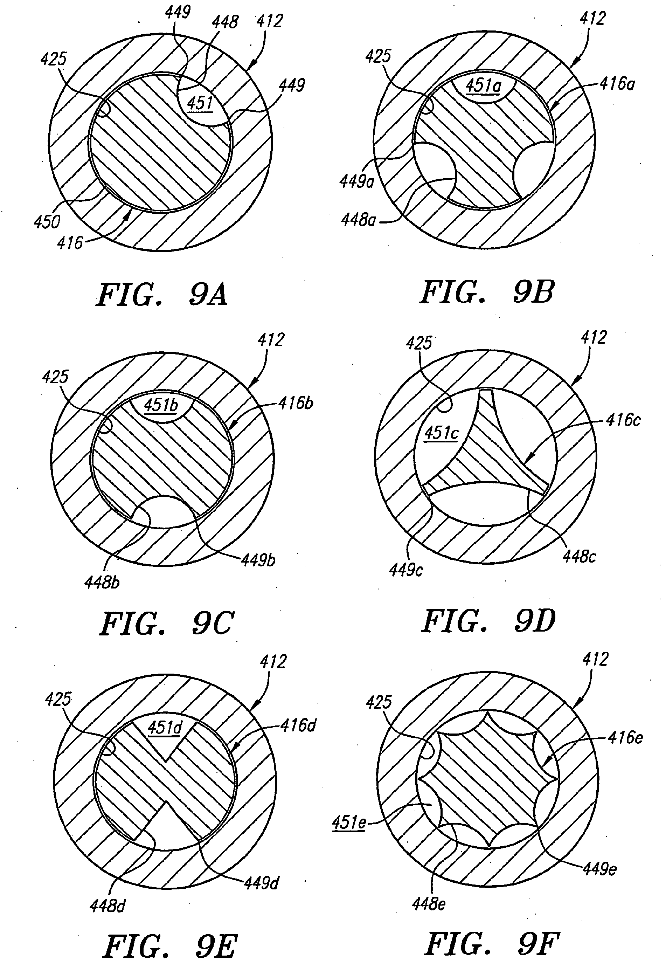

Preferably, as shown in FIG. 9A, the lumen 424 in the plug member 412 has a cross-section, e.g., diameter, similar to that of the guide member 416 such that edges 449 of the groove 448 slidably engage an inner wall 425 of the plug member 412. Thus, the plug member 412 and guide member 416 may together define a lumen 451, which is generally referred to as a bleed back lumen (although it may receive other fluids in addition to or instead of blood). Similarly, the lumen 440 in the carrier member 414 may also have a cross-section similar to and aligned with the lumen 424 in the plug member 412, thereby further defining the bleed back lumen 451.

In alternative embodiments, shown in FIGS. 9B-9F, the guide member 416ₐ-416ₑ may have one of a variety of cross-sections, thereby providing one or more bleed back lumens 451ₐ-451ₑ defined by edges 449ₐ-449ₑ of one or more respective grooves 448ₐ-448ₑ in the guide member 416ₐ-416ₑ and an inner wall 425 of the plug member 412. In addition, the grooves 448 may have one of a variety of shapes, such as the exemplary concave, notched, or flat cross-sections shown in FIGS. 9B-9F. Thus, any number of bleed back lumens, having any desired shape may be provided in the apparatus 410.

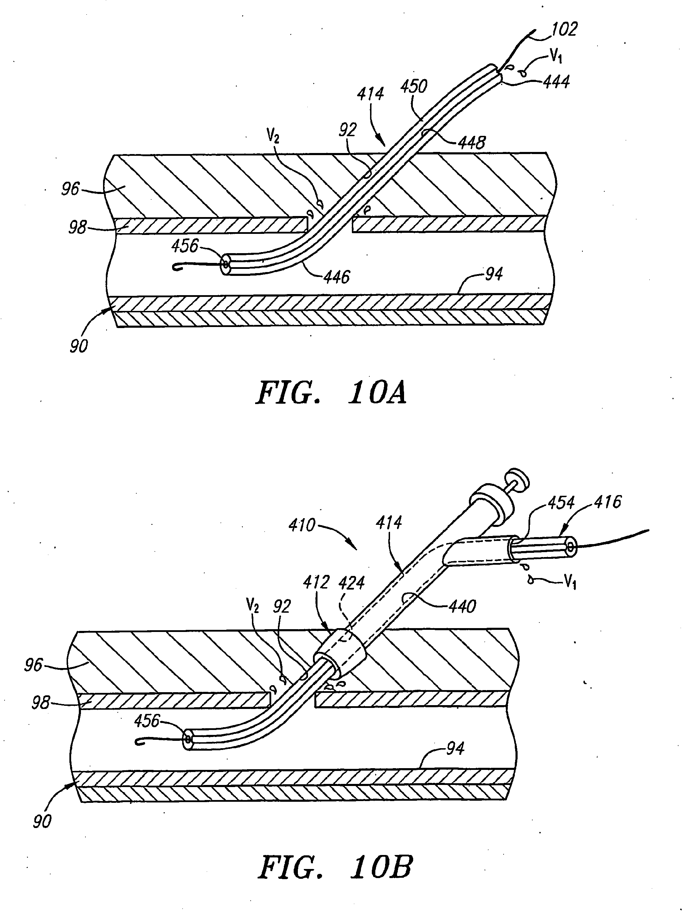

Turning to FIGS. 10A-10D, during use, the apparatus 410 may be used to seal and/or close a passage through tissue 96, such as a puncture 92 communicating with a blood vessel 90 or other body lumen. The puncture 92 may be used to provide percutaneous access through a wall 98 of the vessel 90 into the vessel lumen 94. For example, the puncture 92 may facilitate performing an endovascular procedure within a patient's vasculature, such as angioplasty, stenting, atherectomy, and the like, or may otherwise provide access via the vessel 90 to a region within the patient's body.

Upon completion of the procedure, any instruments, such as an introducer sheath (not shown), may be removed from the vessel 90 and puncture 92. If a guidewire 102 is used during the procedure, the guidewire 102 may be removed before delivering the plug member 412. Alternatively, however, the guidewire 102 may be used to guide the plug member 412 into position, as described below. In a further alternative, an introducer sheath (not shown), such as that used to previously introduce instruments into the vessel 90 may be left in place, and the guidewire 102 may be removed.

Initially, the guide member 416 is introduced into the puncture 92, for example, by inserting the distal tip 446 of the guide member 416 into the puncture 92 and advancing the distal tip 446 until it enters the vessel 90, as shown in FIG. 10A. If the guidewire 102 is in place, generally as shown, the guidewire 102 may be backloaded into a guidewire lumen 456 in the guide member 416 in a conventional manner before inserting the distal tip 446 into the puncture 92. Alternatively, if an introducer sheath is left in place, the guide member 416 may be advanced through a lumen of the introducer sheath.

As the distal tip 446 enters the vessel 90 (e.g., over the guidewire 102 or through an introducer sheath), capillary action may cause a relatively small amount of blood, represented by v₁, to travel proximally along the groove 448 to the proximal end 444 of the guide member 416. Because the groove 448 does not provide a substantially enclosed lumen, however, much (and preferably most) of the blood entering the puncture 92 may be dispersed within subcutaneous tissue, e.g., adjacent to the wall 98 of the vessel 90, as represented by v₂, and not travel to the proximal end 444 of the guide member 416. If a guidewire and/or an introducer sheath is used to direct the guide member 416 into the vessel 90, they may be removed once the distal tip 436 is positioned within the vessel 90.

The plug member 412 may then be advanced over the guide member 416 into the puncture 92, as shown in FIG. 10B. Preferably, the proximal end 446 of the guide member 416 is inserted into the lumen 424 in the plug member 412. The plug member 412 is then advanced over the guide member 416 and into the puncture 92 until the proximal end 446 of the guide member 416 passes through the lumen 440 in the carrier member 414 and exits the lumen 440 at the proximal end 436 of the carrier member 414. Optionally, the carrier member 414 may not include a lumen and a separate lumen member (not shown) may be provided that communicates with the lumen 424 in the plug member 412.

Preferably, the lumens 424, 440 in the plug member 412 and/or the carrier member 414 have a cross-section, e.g., diameter, similar to that of the guide member 416 such that the groove 448 and the lumens 424, 440 define a bleed back lumen 451, as described above. Thus, as the plug member 412 is advanced over the guide member 416, the bleed back lumen 451 is also advanced distally.

If the plug member 412 includes a thread pattern 418 (as shown in FIG. 8B), the carrier member 414 may be rotated in a first direction to thread the plug member 412 into the puncture 92. Because the plug member 412 is coupled to the carrier member 414, as the carrier member 414 is rotated, the plug member 412 is rotated and threaded into the puncture 92 towards the vessel 90. Consequently, the outer surface 430 and thread pattern 418 of the plug member 412 may engage tissue 96 surrounding the puncture 92, thereby substantially sealing the puncture 92 from fluids, such as blood, within the vessel 90.

One advantage of providing an external thread on the plug member 412 is that the thread pattern 418 may facilitate advancement of the plug member 412 through layers of tissue (not shown) towards the vessel 90. For example, the tissue 96 may include one or more intermediate layers of fascia or other tissue structures disposed between layers of fat (also not shown). The fascia layer may be relatively thin, yet resilient and tough, and therefore it may be difficult to advance a device axially through the layer without deflecting the layer towards the vessel 90.

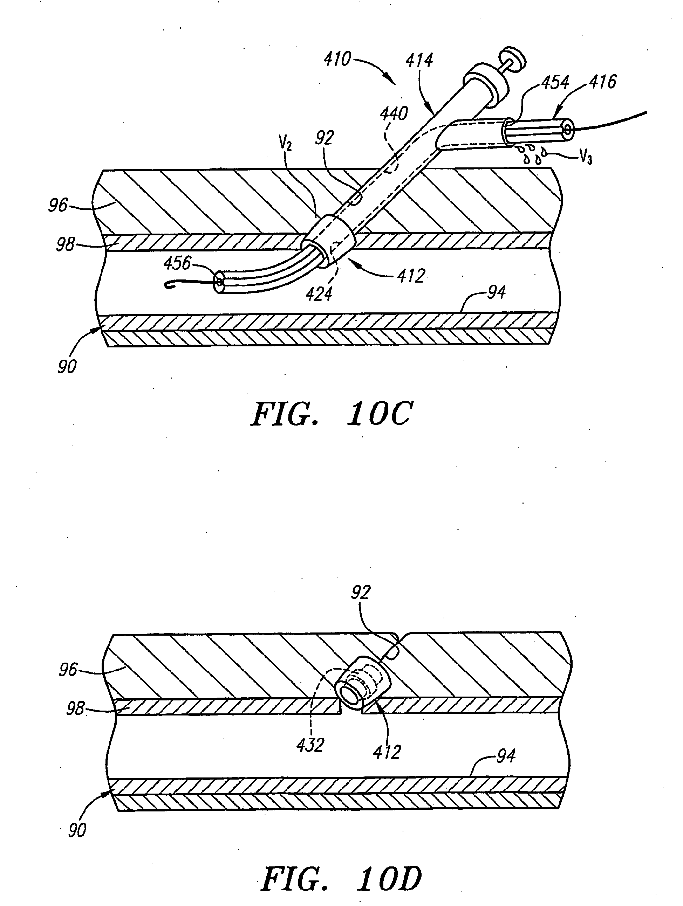

When the plug member 412 is advanced through the wall 98 of the vessel 90, as shown in FIG. 10C, the bleed back lumen 451 may become exposed to fluid, e.g., blood, within the vessel 90. Because of internal blood pressure, the fluid may enter the bleed back lumen 451 (not shown, see FIG. 8A), pass through the lumens 424, 440 in the plug member 412 and the carrier member 414, and exit the proximal port 454 of the carrier member 414, as represented by v₃, thereby providing a visual indication that the vessel 90 has been attained. If a relatively low rate of fluid flow was previously observed, a substantially higher rate of fluid flow may be observed, because fluid entering the puncture 92 may no longer be dispersed within the subcutaneous tissue, but pass instead through the bleed back lumen 451. This visual indication from the proximal port 454 may provide feedback that the distal end 422 of the plug member 412 has reached the vessel 90, as will be appreciated by those skilled in the art.

If desired, the carrier member 414 may then be withdrawn partially to move the plug member 412 to a predetermined deployment depth, e.g., offset proximally form the wall 98 of the vessel 90. For example, bleed back may occur when the plug member 412 is within or in close proximity to the vessel 90, as shown in FIG. 10C. If the plug member 412 includes an external thread, rotation of the apparatus 410 may be reversed, i.e., in a second direction opposite the first direction, to withdraw the plug member 412 a predetermined distance relative to the vessel 90. If no external thread is provided, the carrier member 414 may simply be pulled proximally. Otherwise, the plug member 412 may be left in place, e.g., in contact with the wall 98 of the vessel 90.

Once the desired deployment location is attained, the plug member 412 may be released from the carrier member 414, e.g., by depressing actuator 43. The guide member 416 may be withdrawn from the plug member 412 and carrier member 414 either before or after releasing the plug member 412. The sealing member 432 preferably substantially seals the lumen 424 within the plug member 412 thereafter to prevent fluid within the vessel 90 from passing therethrough to leak from the puncture 92.

For example, as explained above, the sealing member 432 may include a material that expands when exposed to fluid. As the guide member 416 is withdrawn, fluid, e.g., blood, may flow proximally through the lumen 424 in the plug member 412, e.g., until it encounters the sealing member 432. Although a relatively small amount of fluid may pass beyond the sealing member 432, the sealing member 432 may expand relatively quickly due to the fluid contact until it substantially seals the lumen 424. Alternatively, the sealing member 432 may be a valve that may automatically open to accommodate the guide member 416, but may automatically close upon withdrawal of the guide member 416. In a further alternative, the sealing member 432 may be manually actuated by the user.

Finally, as shown in FIG. 10D, the carrier member 414 and the guidewire 102 (if still in place) may be withdrawn, leaving the plug member 412 in place to substantially seal the puncture 92. If the plug member 412 is bioabsorbable, it may remain within the puncture 92 as the tissue heals, thereby allowing the wall 98 of the vessel 90 and tissue 96 surrounding the passage 92 to at least partially heal before the plug member 412 is absorbed. Alternatively, the plug member 412 may be retrieved once the tissue between the plug member 412 and the vessel 90 has substantially healed, as described in the applications incorporated above.

Turning to FIGS. 11A-11B, yet another embodiment of an apparatus 510 is shown that includes a plug member 512 and a handle device 514. The plug member 512 includes a body, preferably formed from bioabsorbable material, including a proximal end 520 and a distal end 522. An outer surface 530 of the plug member 512 may be substantially smooth or may include a helical thread pattern (not shown) extending along the outer surface 530 at least partially between the proximal and distal ends 520, 522, similar to the embodiment described above.

In addition, the plug member 512 includes a lumen 524 that extends between a proximal opening 526 and a distal opening 528 generally parallel to a longitudinal axis 538. An inner lip 525 is provided within the lumen 524, e.g., adjacent the distal end 522 of the plug member 512, which may be a continuous annular ridge or a plurality of tabs.

A sealing member 532 is disposed within the lumen 524 in an open position (shown in FIG. 11A) adjacent the inner lip 525. The sealing member 532 is a generally annular-shaped member, preferably a coil of material including one or more overlapping layers, which may be formed from a biocompatible, and preferably a bioabsorbable material, similar to the plug member 512. Alternatively, the sealing member 532 may be an enclosed ring that may be formed from semi-rigid or flexible material. In the open position, the lumen 524 is substantially open, i.e., the sealing member 532 does not generally obstruct the lumen 524. The sealing member 532 is compressible distally against the lip 525 to become compressed or wedged therein (as shown in FIG. 11B), thereby defining a closed position for substantially sealing the lumen 524 from fluid flow therethrough.

The handle device 514 includes an outer carrier tube 534, and an inner delivery tube 536, the inner tube 536 being coaxially and/or slidably disposed within the outer tube 534. The handle device 514 may include a handle and/or actuator (not shown) on a proximal end of the handle device 514 for manipulating the handle device 514 and/or for controlling movement of the inner tube 536 relative to the outer tube 534. A distal end 540 of the outer tube 534 may be received in an annular recess 527 to couple the plug member 512 to the handle device 514. The distal end 540 may frictionally engage a wall of the recess 527, e.g., providing a desired resistance to removing the distal end 540 from the recess 527. In addition or alternatively, the distal end 540 and/or the plug member 512 may include one or more detects or connectors (not shown), similar to the embodiment described above.

A distal end 542 of the inner tube 536 preferably has a size for being slidably received within the lumen 524 and substantially abutting the sealing member 532. The inner tube 536 preferably includes a lumen 544 that communicates with the lumen 524. Thus, the lumens 544, 524 may provide a lumen for receiving a guide member (not shown), such as that shown and described in connection with FIG. 7A.

Use of the apparatus 510 proceeds similar to the previous embodiment, except that the sealing member 532 is manually activated, rather than being activated automatically upon fluid contact. The apparatus 510 is assembled by inserting the distal end 540 of the outer tube 534 into the recess 527 and the distal end 542 of the inner tube 536 into the lumen 524, as shown in FIG. 11A. A guide member (not shown) may be directed into a passage communicating with a vessel, e.g., through an introducer sheath, which is then removed once a distal end of the guide member is positioned within the vessel, similar to the previous embodiment.

The plug member 512 is inserted into the passage over the guide member until the distal end 522 of the plug member 512 enters the vessel. As the plug member 512 enters the vessel, a bleed back lumen defined by a groove (not shown) in the guide member and the wall of the lumen 524 becomes exposed to the interior of the vessel, causing fluid to flow into the bleed back lumen to a proximal outlet port or other bleed back indicator (not shown) on the handle device 514.

The inner tube 536 may then be advanced distally relative to the outer tube 534, thereby engaging the sealing member 532 and forcing it distally against the inner lip 525. Because of its inherent flexibility and/or because of its coil shape, the sealing member 532 may compress and/or become crushed, wedging the sealing member 532 within the lumen 524 and sealing the lumen 524 from fluid flow therethrough, as shown in FIG. 11B. In addition, a distal portion of the sealing member 532 may attached to an inner wall of the plug member 512 defining the lumen 524, e.g., by an adhesive, sonic welding, and the like. Alternatively, if the sealing member 532 is attached to the inner wall, the lip may be eliminated (not shown).

The plug member 512 may be released from the handle device 514, e.g., by withdrawing the outer tube 536 and/or by releasing connectors securing the plug member 512 to the distal end 540 of the outer tube 534. The handle device 514 may then be withdrawn form the passage, leaving the plug member 512 in place, similar to the embodiment described above.

Turning to FIG. 12, another embodiment of an apparatus 610 is shown that includes a plug member 612 and a handle device 614. The plug member 612 includes a tapered body, preferably formed from bioabsorbable material, including a proximal end 620 and a distal end 622. Preferably, the plug member 612 is continuously tapered from the proximal end 620 towards the distal end 622, although alternatively, only a distal portion of the plug member 612 may be tapered (not shown). The plug member 612 may have a substantially constant taper or the taper rate may vary along the length of the plug member 612 (not shown). The distal end 622 may be substantially rounded or blunt, may include a concave distal tip as shown, and/or may be pointed (not shown).

In addition, the plug member 612 includes a lumen 624, and may include a sealing member 632 disposed within the lumen 624, similar to the embodiments described above. The lumen 624 communicates with a distal port 628 that is located on or adjacent the distal end 622 of the plug member 612.

A helical thread pattern 618 extends along an outer surface 630 of the plug member 612 at least partially between the proximal and distal ends 620, 622. Alternatively, the outer surface 630 may be substantially smooth or may include gripping elements for allowing distal advancement but preventing proximal withdrawal of the plug member 612 from within a passage through tissue, similar to the embodiments described above.

The handle device 614 is an elongate member having a proximal end 634, a distal end 636, and a lumen 640 extending therebetween. Preferably, the plug member 612 and the handle device 614 include cooperating elements (not shown) for coupling the plug member 612 to the handle device 614, i.e., to prevent rotation of the plug member 612 relative to the handle device 614. For example, cooperating connectors (not shown) may be provided for releasably securing the plug member 612 to the distal end 636 of the handle device 614, similar to the embodiment described above. If the plug member 612 has an internal sealing member (not shown), the handle device 614 may include an internal member, e.g., inner tube (also not shown), for wedging or otherwise activating the sealing member.

Turning to FIGS. 13A-13B, during use, the apparatus 610 may be used to seal and/or close a passage through tissue 96, such as a puncture 92 used to access a blood vessel 90 during an endovascular procedure, similar to the embodiment described above. Upon completion of the procedure, any instruments, such as an introducer sheath, a guidewire, and the like (not shown), may be removed from the vessel 90 and puncture 92.

As shown in FIG. 13A, the distal end 622 of the plug member 612 may be inserted into the puncture 92, and the handle device 614 rotated in a first direction to advance the plug member 612 into the puncture 92. Because the plug member 612 is coupled to the handle device 614, rotation of the handle device 614 preferably threads the plug member 612 into the puncture 92 towards the vessel 90. Consequently, the outer surface 630 and/or the thread pattern 618 of the plug member 612 may engage tissue 96 surrounding the puncture 92, thereby substantially sealing the puncture 92 from fluids, such as blood, within the vessel 90. In addition, the thread pattern 618 may facilitate advancing the plug member 612 through any intervening layers of tissue, e.g., one or more layers of fascia (not shown), similar to the embodiments described above.

As shown in FIG. 13B, when the distal end 622 of the plug member 612 is advanced through the wall 98 of the vessel 90, the distal port 628 becomes exposed to fluid, e.g., blood, within the vessel 90. Because of internal blood pressure, the fluid may enter the distal port 628 and pass through the lumens 624, 640, thereby providing a visual indication that the vessel 90 has been attained, similar to the embodiment described above.

The plug member 612 may then be released from the handle device 614, e. g., by activating an actuator (not shown, see FIG. 12) on the proximal end of the, handle device 614, and the handle device 614 withdrawn from the passage 92. Preferably, the distal end 622 of the plug member 612 does not extend substantially into the vessel 90, thereby minimizing obstruction of the lumen 94. The tapered profile of the plug member 612 may substantially anchor the plug member 612 within the passage 92 and/or enhance sealing the passage 92 from fluid flow.

Alternatively, after bleed back indicates that the distal end 622 is located within the vessel 90, rotation of the handle device 614 may be reversed, i. e., in a second direction opposite the first direction, to withdraw the plug member 612 a predetermined distance relative to the vessel 90. Once the desired deployment location is attained, the plug member 612 may be released from the handle device 614. The handle device 614 may be withdrawn, leaving the plug member 612 in place to substantially seal the passage 92.

As fluid flows through the lumen 624, the sealing member 632 may expand to substantially seal the lumen 624 to prevent further fluid within the vessel 90 from passing therethrough to leak from the puncture 92. Alternatively, the lumen 624 may be sufficiently small that the sealing member 632 may be eliminated, and may seal as hemostasis occurs. In a further alternative, the sealing member 632 may be manually wedged or otherwise closed to seal the lumen 624.

If the plug member 612 is bioabsorbable, it may remain within the puncture 92 as the tissue heals, thereby allowing the wall 98 of the vessel 90 and tissue 96 surrounding the passage 92 to at least partially heal before the plug member 612 is absorbed. Alternatively, the plug member 612 may be retrieved once the tissue between the plug member 612 and the vessel 90 has substantially healed.

An apparatus in accordance with the present invention may be used for procedures other than a surgical procedure. For example, an apparatus in accordance with the present invention may be used to seal temporarily or indefinitely other passages, such as wounds, punctures, and the like that extend through tissue. The apparatus may be used in an emergency setting, e. g., to seal a bullet wound or other puncture temporarily until the patient may be subsequently treated and/or transported to a location where conventional surgery or other treatment may be performed.

The apparatus may be removably introduced into the passage to seal it and/or may be deployed within the passage. A bleed back lumen, such as that described above, may be used to position the apparatus. Alternatively, flow through a blood vessel or other body passage proximate the passage may be monitored to position the plug member relative to the vessel, similar to the methods described above.

Turning to FIGS. 14A, 14B, and 15, another preferred embodiment of an apparatus 710 for sealing a passage through tissue, in accordance with the present invention. Generally, the apparatus 710 includes a plug member 712, an elongate shaft or handle device 714, and an obturator 716.

The plug member 712 is a substantially rigid body, preferably having a generally cylindrical shape, including a proximal end 720, a distal end 722, and an outer surface 730. The plug member 712 includes a lumen 724 that extends between a proximal opening 726 and a distal opening or port 728..

The plug member 712 may be formed from a biocompatible material, e.g., a plastic, such as polyethylene or polyester. Preferably, the plug member 712 is formed at least partially (and more preferably entirely) from bioabsorbable material, such as collagen, polyglycolic acids (PGA's), polyactides (PLA's), and the like, that may be at least partially absorbed by the patient's body over time. Alternatively, the plug member 712 may be a semi-rigid or flexible body or may have a substantially flexible distal tip (not shown), e.g., to facilitate atraumatic insertion of the plug member 712 into the passage. In addition or alternatively, the plug member 712 may be tapered along its length, and/or the distal end 722 may be rounded to facilitate advancement of the plug member 712 into a passage through tissue.

In a preferred embodiment, the plug member 712 has a length of not more than about ten millimeters (10 mm), and more preferably between about one and ten millimeters (1-10 mm). The plug member 712 also preferably has a diameter of between about one and twenty millimeters (1-20 mm). Preferably, the length and diameter have a ratio that not more than about two-to-one.

The plug member 712 generally includes a helical thread pattern 718, including one or more helical threads, that extend at least partially between its proximal and distal ends 720, 722. Preferably, the thread pattern 718 extends completely to the distal end 722 of the plug member 712, and may be tapered at the distal end 722 to facilitate introduction into a passage through tissue (not shown). The helical thread 718 is preferably substantially rigid and may have a substantially square cross-section to facilitate sealing of a passage into which the plug member 712 is threaded. In a preferred embodiment, the helical thread 718 is integrally formed on the outer surface 730 of the plug member 712. For example, the plug member 712 and thread718 may be formed by injection molding. Alternatively, the threads may be cut or otherwise formed in the outer surface 730 of the plug member 712.

As best seen in FIG. 15 (in which the handle device 714 has been eliminated for convenience), a sealing member 732 is provided within the lumen 724 for substantially sealing the lumen 724 from fluid flow therethrough. In a preferred embodiment, the sealing member 732 has an annular shape, and is mounted within an annular recess 733 in the lumen 724. The sealing member 732 is preferably formed from a material that expands when exposed to fluids, e. g., an expandable foam. More preferably, the sealing member 732 is also bioabsorbable, similar to the plug member 712 itself. Exemplary materials that may be appropriate for use in the sealing member 732 and/or for the plug member 712 are disclosed in U.S. Patent No. 6,924,630. Alternatively, the sealing member 732 may be a valve (not shown) that is biased to substantially seal the lumen 724 from fluid flow, but may be opened to facilitate introduction of one or more devices, e. g., the obturator 716 therethrough, as described embodiment, the plug member 712 may include a cavity (not

In an alternative embodiment, the plug member 712 may include a cavity (not shown) in the distal end 722. A material (also not shown) may be provided in the cavity, such as extra-cellular matrix material, e. g., intestinal, stomach, or bladder submucosa, collagen, an infection-resistant material, and the like, that may promote hemostasis and/or healing of the tissue. Alternatively, such material may be otherwise detachably secured to the distal end 722 of the plug member 712, either within a cavity or across the distal end 722 without a cavity. For example, the material may be secured using a biodegradable adhesive or a mechanical fastener, such as one or more clips (not shown).

Returning to FIGS. 14A and 14B, the handle device 714 has a proximal end 734 and a distal end 736, and defines a longitudinal axis 738 that extends between the proximal and distal ends 34,736. A lumen 740 also extends between the proximal and distal ends 734,736, e. g., for accommodating insertion of the obturator 716 therethrough. A handle 742 may be provided on the proximal end 734 of the handle device 714 for facilitating manipulation of the apparatus 710, e.g., to facilitate rotation of the apparatus710 into a passage, as described below.

Preferably, the handle device 714 is a substantially rigid tubular member, formed from a biocompatible material, e. g., plastic, such as polyethylene or polyester, or metal, such as stainless steel. The handle device 714 preferably has a cross-section that is substantially smaller than a cross-section of the plug member 712, e. g., to minimize dilation of a passage into which the apparatus710 is inserted.

At least one of the plug member 712 and the distal end 736 of the handle device 714 include a connector. Preferably, the plug member 712 and the distal end 736 of the handle device 714 include cooperating connectors (not shown) for releasably securing the plug member 712 to the handle device 14, as described in US Patent No. 6,846,319. Preferably, the cooperating connectors substantially couple the plug member 712 to the handle device 714 such that the plug member 712 cannot move independently of the handle device 714, e. g., such that the plug member 712 may be rotated only by rotating the handle device 714.

For example, the plug member 712 may include a recess (not shown) in its proximal end 720 and the handle device 714 may include a mechanism, e. g., a frame and/or radially projecting fingers (not shown), for frictionally engaging the wall of the recess. Alternatively, the recess may include slots for positively receiving the mechanism on the handle device 714. In a further alternative, the plug member 712 may include a hub (not shown) extending from its proximal end 720 and the handle device 714 may include a mechanism for detachable securing the hub to the handle device 714.

Preferably, the handle 742 includes an actuator (not shown) that may be activated to release the connectors securing the plug member 712 to the handle device 14. For example, the actuator may include a button coupled to a control rod or wire (not shown) that extends through the handle device 714 to its distal end 36. Upon depression of the button, the control rod may be moved, thereby disengaging the connector on the handle device 714 from the mating connector on the plug member 712. In another alternative, the distal end 736 of the handle device 714 and the plug member 712 may include mating threads (not shown) so that the handle device 714 may be rotated with respect to the plug member 712 to release the plug member 712. In this embodiment, the mating threads should wind helically in the same direction as the thread pattern 718 on the plug member 712 to ensure hat the plug member 712 is not released prematurely from the handle device 714.

The obturator 716 is an elongate member, preferably having a proximal end 744 and a substantially atraumatic and/or flexible distal tip 746. An inlet or bleed back port 748 is provided on the distal tip 746, and a bleed back lumen 750 extends from the inlet port 748 to the proximal end 744. The proximal end 744 may include an outlet port 752, which may include any conventional structure for detected or observing fluid passing from the back bleed lumen 750.

The obturator 716 has a size and shape for insertion through the lumen 740 of the handle device 714 and through the lumen 724 of the plug member 12. Once the obturator 716 is fully received through the handle device 14, the distal tip 46 of the obturator716 may extend beyond the distal end 722 of the plug member 712, as shown in FIG. 14B. The obturator 716 and the handle device 714 may include cooperating detents (not shown) for securing the obturator 716 once it is fully received through the handle device 714 and plug member 712. The detents may substantially permanently or releasably couple the obturator 716 to the handle device 714.

In alternative embodiments, an expandable member (not shown) may be provided on or adjacent the distal tip 746 of the obturator 716, in addition to the bleed back port and lumen 748,750. The expandable member may be expandable, e. g., when the distal tip is disposed within a body lumen, for providing tactile feedback of a location of the distal end of the plug member with respect to the body lumen. The expandable member may be a balloon, one or more expandable wings, such as those disclosed in US Patent No. 6,780,197, or a helical tether device.

Turning to FIGS.16A-16D, during use, the apparatus 710 may be used to seal and/or close a passage through tissue 96, such as a puncture 92 communicating with a blood vessel90 or other body lumen. The puncture 92 may be used to provide percutaneous access to the vessel 90. For example the puncture 92 may facilitate performing an endovascular procedure within a patient's vasculature, such as angioplasty, stenting, atherectomy, and the like, or may otherwise provide access via the vessel 90 to a region within the patient's body.

Upon completion of the procedure, any instruments, such as an introducer sheath (not shown), may be removed from the vessel 90 and puncture 92. If a guidewire 102 is used during the procedure, the guidewire 102 may be removed before delivering the plug member 712, or preferably, the guidewire 102 may be used to guide the plug member 712 into position, as described below.

Initially, the apparatus 710 is assembled as shown in FIG. 14B, i.e., the plug member 712 is connected to the handle device 714, and the obturator 716 is inserted through the handle device 714 and plug member 712. The apparatus 710 may then be introduced into the puncture 92, for example, initially by inserting the distal tip 746 of the obturator 716 into the puncture 92. If the guidewire 102 is in place, generally as shown, the guidewire 102 may be backloaded into a guidewire lumen (not shown) in the obturator 716 in a conventional manner before inserting the distal tip 746 into the puncture 92.

As the obturator 716 is advanced into the puncture 92 (e.g., over the guidewire 102), the plug member 712 is inserted into the puncture 92, as shown in FIG. 16A. Because of the thread pattern 718, the handle device 714 is then rotated in a first direction to thread the plug member 712 into the puncture 92. Consequently, the outer surface 730 and thread pattern 718 engage tissue 96 surrounding the puncture 92, thereby substantially sealing the puncture 92 from fluids, such as blood, within the vessel 90. The apparatus 710 may then be rotated in a first direction about its longitudinal axis 738 to thread the plug member 712 substantially atraumatically deeper into the puncture 92.

Turning to FIG. 16B, as the plug member 712 is advanced, the thread pattern 718 may facilitate advancement of the plug member 712 through layers of tissue towards the vessel 90. For example, the tissue 96 may include one or more intermediate layers of fascia 99 or other tissue structures disposed between layers of fat. The fascia layer 99 may be relatively thin, yet resilient and tough, and therefore it may be difficult to advance a device axially through the layer 99 without deflecting the layer 99 towards the vessel 90. Because of the thread pattern 718, a plug member 712 in accordance with the present invention may be threaded or screwed through the fascia layer 99 towards the vessel 90. This may substantially reduce the risk of the fascia layer 99 being deflected towards the vessel 90 as the plug member 712 is advanced towards the vessel 90, thereby minimize deflection of the plug member 712 away from the vessel 90 once released within puncture 92 that may otherwise occur if the fascia layer 99 is compressed towards the vessel 90.

When the plug member 712 is advanced into the puncture 92, the distal tip 746 of the obturator 716 eventually passes through the wall 98 of the vessel 90, whereupon the bleed back port 748 becomes exposed to fluid, i.e., blood, within the vessel 90. Because of internal blood pressure, the fluid enters the bleed back port 748, passes through the bleed back lumen 750 (not shown in FIGS. 16A-16D), and exits the outlet port 752 (as represented by drops 754), thereby providing a visual indication that the vessel 90 has been attained. Because of the relative lengths of the plug member 712, the handle device 714, and the obturator 716, this visual indication provides feedback on the location of the distal end 722 of the plug member 712 relative to the vessel 90, as will be appreciated by those skilled in the art. The relative lengths may be predetermined such that the plug member 712 is at a preferred deployment depth when the bleed back indication is observed. For example, the deployment depth may place the plug member 712 in close proximity to the vessel 90, e.g., without exposing the distal end 722 within the vessel 90.

Preferably, the relative lengths may be such that the apparatus 710 needs to be counter-rotated to attain the preferred deployment depth. For example, bleed back may occur when the plug member 712 is within or in close proximity to the vessel 90, as shown in FIG. 16B. Rotation of the apparatus 710 may then be reversed, i.e., in a second direction opposite the first direction, to withdraw the plug member 712 a predetermined distance relative to the vessel 90, as shown in FIG. 3C. Because of the known pitch of the thread pattern 718, the distance that the plug member 712 is moved relative to the vessel 90 may be related directly to the number of rotations and/or partial rotations that the apparatus 710 is counter-rotated.

Once the desired deployment location is attained, the plug member 712 may be released from the handle device 714. The obturator 716 may be withdrawn from the plug member 712 and handle device 714 either before or after releasing the plug member 12. The sealing member 732 (not shown, see FIG. 15) preferably substantially seals the lumen (not shown, see FIG. 15) within the plug member 712 to prevent fluid within the vessel 90 from passing therethrough to leak from the puncture.

Preferably, as explained above, the sealing member 732 is a material that expands when exposed to fluid. For example, as the obturator 716 is withdrawn, fluid, e.g., blood, may flow proximally through the lumen 724 in the plug member 712, e.g., until it encounters the sealing member 732. Although a relatively small amount of fluid may pass beyond the sealing member 732, the sealing member 732 may expand substantially due to the fluid contact until it substantially seals the lumen. Alternatively, the sealing member 732 may be a valve that may open to accommodate the obturator 716, but may automatically close upon withdrawal of the obturator 716.

Finally, as shown in FIG. 16D, the handle device 714 and the guidewire 102 (if still in place) may be withdrawn, leaving the plug member 712 in place to substantially seal the puncture 92. If the plug member 712 is bioabsorbable, it may remain within the puncture 92 as the tissue heals, thereby allowing the wall 98 of the vessel 90 and tissue 96 surrounding the passage 92 to at least partially heal before the plug member 712 is absorbed. Alternatively, the plug member 712 may be retrieved once the tissue between the plug member 712 and the vessel 90 has substantially healed, as described in the application incorporated above.

Turning to FIGS. 17A and 17B, another embodiment of an apparatus 810 is shown that includes a plug member 812 and a handle device 814. The plug member 812 includes a body, preferably formed from bioabsorbable material, including a proximal end 820 and a distal end 822. A helical thread pattern 818 extends along an outer surface 830 of the plug member 812 at least partially between the proximal and distal ends, similar to the embodiment described above. In addition, the plug member 812 includes a lumen 824 and a sealing member 832 disposed within the lumen 824, similar to the embodiment described above.

The handle device 814 is an elongate member having an enlarged portion 834 and a reduced portion 836 defining a shoulder 838 therebetween. The enlarged portion 834 may include a handle 842 on the proximal end 840. The reduced portion 836 has a size for insertion through the lumen 824 of the plug member 812 and terminates in a distal tip 846 that may be substantially atraumatic and/or flexible, similar to the obturator distal tip described above. A bleed back port 848 is provided in the distal tip 846 that communicates with a bleed back lumen 850 that extends to an outlet port 852 in the proximal end 840.

As shown in FIG. 17B, the reduced portion 836 may be inserted into the lumen 824 until the distal tip 846 extends beyond the distal end 822 of the plug member 812 and/or the shoulder 838 abuts the proximal end 820 of the plug member 812. Alternatively, the shaft of the handle device 814 may have a substantially uniform cross-section, similar to the reduced portion 834, and a raised portion (not shown) may be provided on the shaft, e.g., an annular ridge against which the plug member 812 may abut. Thus, the shoulder 838 or other raised portion may limit proximal movement of the plug member 812 relative to the handle device 814.

Preferably, the plug member 812 and the handle device 814 include cooperating elements (not shown) for coupling the plug member 812 to the handle device 814, i.e., to prevent rotation of the plug member 812 relative to the handle device 814. For example, all or a portion of the reduced portion 836 of the handle device 814 may have a noncircular cross-section, and all or a mating portion of the lumen 824 may have a complementary noncircular cross-section. Alternatively, cooperating longitudinal slots and tabs and the like may be provided on the reduced portion 836 and within the lumen 824 of the plug member 814. Thus, when the reduced portion 836 is fully inserted through the lumen 824, rotation of the plug member 812 may be coupled to rotation of the handle device 814. In a further alternative, the plug member 812 and the handle device 814 may include connectors that may releasably couple the plug member 812 to the handle device 814, similar to the embodiment described above.

The apparatus 810 may be used to seal and/or close a passage through tissue, such as a puncture communicating with a blood vessel or other body lumen (not shown), similar to the embodiment described above. Upon completion of a procedure accessed via the puncture, any instruments may be removed from the vessel and puncture, although a guidewire (not shown) may remain, similar to the embodiment described above. The apparatus 810 may be assembled as shown in FIG. 17B, i.e., with reduced portion 834 of the handle device 814 fully inserted into the plug member 812. The distal tip 846 may be inserted into the puncture, e.g., over a guidewire (not shown) if still in place within the puncture, until the plug member 812 is inserted into the puncture 92.

The apparatus 810 may then be rotated to thread the plug member 812 into the puncture such that the outer surface 830 and thread pattern 818 engage tissue surrounding the puncture to substantially seal the puncture. When the distal tip 846 enters the vessel, the bleed back port 848 becomes exposed to blood within the vessel. Because of internal blood pressure, fluid within the vessel enters the port 848, passes through the bleed back lumen 850, and exits the outlet port 852, thereby providing a visual indication that the vessel has been attained.

If desired, rotation of the apparatus 810 may then be reversed to withdraw the plug member 812 a predetermined distance relative to the vessel. The plug member 812 may then be released from the handle device 814. The handle device 814 may then be withdrawn from the plug member 812 (and the guidewire, if still present). Preferably, the reduced portion 834 of the handle device 814 may simply be withdrawn from within the lumen 824, without requiring disengagement of connectors, which may simplify construction of the handle device 814 compared to the embodiment described above. As the reduced portion 834 is withdrawn from the lumen 824, the sealing member 832 becomes exposed to fluid passing through the lumen 824. Preferably, as explained above, the sealing member 832 expands when exposed to the fluid to substantially seal the lumen 824 from subsequent fluid flow. Alternatively, the sealing member 832 may be a valve or an element that may controllably opened or closed (not shown).

Turning to FIG. 18, yet another embodiment of an apparatus 910 is shown that includes a plug member 912 and a handle device 914. The plug member 912 includes a body, preferably formed from bioabsorbable material, including a proximal end 920, a distal end 922, and a helical thread pattern 918 that extends along an outer surface 930 at least partially between the proximal and distal ends 920, 922, similar to the embodiment described above. In addition, the plug member 912 includes a lumen 924 including an inlet or bleed back port 248 and a sealing member (not shown) disposed within the lumen 924. Alternatively, the lumen 924 may have a sufficiently small cross-section that a sealing member may not be necessary.

The handle device 914 has a proximal end 934 and a distal end 936, and defines a longitudinal axis 238 that extends between the proximal and distal ends 934, 936. A lumen 940 also extends between the proximal and distal ends 934, 936. A handle 942 may be provided on the proximal end 934 of the handle device 914 for facilitating manipulation of the apparatus 910, e.g., to facilitate rotation of the apparatus 910 into a passage, similar to the embodiments described above. An outlet port 952 is provided on the proximal end 934, e.g., in the handle 942, that communicates with the lumen 940. Preferably, the handle devices 914 is a substantially rigid elongate shaft formed from biocompatible material. The handle device 914 preferably has a cross-section that is substantially smaller than a cross-section of the plug member 912, similar to the embodiments described above.

The plug member 912 and the distal end 936 of the handle device 914 generally include cooperating connectors (not shown) for releasably securing the plug member 912 to the handle device 914, similar to the first embodiment described above. Preferably, the cooperating connectors substantially couple the plug member 912 to the handle device 914 such that the plug member 912 cannot move independently of the handle device 914, e.g., such that the plug member 912 may be rotated only by rotating the handle device 914.

Preferably, the handle 942 includes an actuator (not shown) that may be activated to release the connectors securing the plug member 912 to the handle device 914. For example, the actuator may include a button coupled to a control rod or wire (not shown) that extends through the handle device 914 to its distal end 936. Upon depression of the button, the control rod may be moved, thereby disengaging the connector on the handle device 914 from the mating connector on the plug member 912.

Before use, the plug member 912 may be coupled to the distal end 936 of the handle device 814. Once the plug member 912 is attached to the distal end 936, the lumen 924 in the plug member 912 communicates with the lumen 940 in the handle device 914.

The apparatus 910 may be used to seal and/or close a passage through tissue, similar to the embodiments described above. After performing a procedure accessed via the puncture, the distal end 936 of the plug member 912 may be inserted into the puncture. The apparatus 910 may then be rotated to thread the plug member 912 deeper into the puncture. When the distal end 936 of the plug member 912 enters the vessel, the bleed back port 948 becomes exposed to blood within the vessel, causing fluid within the vessel to enter the port 948, pass through the lumens 924, 240, and exit the outlet port 952, thereby providing a visual indication that the vessel has been attained.

If desired, rotation of the apparatus 910 may then be reversed to withdraw the plug member 912 a predetermined distance relative to the vessel. The plug member 912 may then be released from the handle device 914. The handle device 914 may then be withdrawn, leaving the plug member 912 in place to substantially seal the puncture.

If the plug member 912 includes a sealing member, the sealing member is exposed to fluid passing through the lumen 924, causing the sealing member to expand when exposed to fluid contact to substantially seal the lumen 924 from subsequent fluid flow. Alternatively, if no sealing member is provided, the lumen may be sufficiently small such that hemostasis may still occur. The lumen 924 may begin to seal on its own or, if necessary, external pressure may be applied to the puncture to promote hemostasis.

Turning to FIGS. 19A-19D, yet another embodiment of an apparatus 1010 is shown that includes a plug member 1012 and a handle device 1014. The plug member 1012 includes a body, preferably formed from bioabsorbable material, including a proximal end 1020 and a distal end 1022. A helical thread pattern 1018 extends along an outer surface 1030 of the plug member 1012 at least partially between the proximal and distal ends 1020, 1022, similar to the embodiments described above.

In addition, the plug member 1012 includes a lumen 1024 that extends between a proximal opening 1026 and a distal opening 1028 generally parallel to a longitudinal axis 1038. The lumen 1024 includes a tapered portion 1025 that tapers towards the distal end 1022. The lumen may include a proximal portion 1024a and a distal portion 1024b on either side of the tapered portion 1025 that may be substantially uniform in cross-section. Thus, the distal opening 1028 may be substantially smaller than the proximal opening 1026, e.g., corresponding to the respective portions of the tapered portion 1025. In addition, the plug member 1012 may include an annular recess 1027 disposed concentrically around the proximal opening 1026.

A sealing member 1032 is disposed in an open position adjacent the wide end of the tapered portion 1025 of the lumen 1024. The sealing member 1032 is a generally annular-shaped member, preferably a coil of material including one or more overlapping layers, which may be formed from a biocompatible, and preferably a bioabsorbable material, similar to the plug member 1012 itself. Alternatively, the sealing member 1032 may be an enclosed ring that may be formed from semi-rigid or flexible material. In its open position, the proximal portion 1024a of the lumen 1024 is substantially open, i.e., the sealing member 1032 does not generally obstruct the lumen 1024. The sealing member 1032 is movable distally into the tapered portion 1025 to become compressed or wedged therein, thereby defining a closed position for substantially sealing the lumen 1024 from fluid flow therethrough.

The handle device 1014 includes an outer carrier tube 1034, and an inner delivery tube 1036, the inner tube 1036 being coaxially and/or slidably disposed within the outer tube 1034. The handle device 1014 may include a handle and/or actuator (not shown) on a proximal end of the handle device 1014 for manipulating the handle device 1014 and/or for controlling movement of the inner tube 1036 relative to the outer tube 1034. A distal end 1040 of the outer tube 1034 may be received in the annular recess 1027 to couple the plug member 1012 to the handle device 1014, The distal end 1040 may frictionally engage a wall of the recess 1027, e.g., providing a desired resistance to removing the distal end 1040 from the recess 1027. In addition or alternatively, the distal end 1040 and/or the plug member 1012 may include one or more connectors (not shown), similar to the embodiments described above.

A distal end 1042 of the inner tube 1036 preferably has a size for being slidably received into the proximal portion 1024a of the lumen 1024. Preferably, when the distal end 1040 of the outer tube 1034 is disposed within the recess 1027, the distal end 1042 of the inner tube 1036 extends into the proximal portion 1024a of the lumen 1024 in close proximity, e.g., contacting, the sealing member 1032. The inner tube 1036 preferably includes a lumen 1044 that communicates with the lumen 1024, more preferably the distal portion 1024b of the lumen 1024. Thus, the lumens 1044, 1024 may provide a bleed back lumen, similar to the embodiments described above.

During use, the apparatus 1010 is assembled by inserting the distal end 1040 of the outer tube 1034 into the recess 1027 and the distal end 1042 of the inner tube 1036 in the proximal portion 1024a of the lumen 1024, as shown in FIGS. 19A and 19B. The plug member 1012 is inserted into a passage communicating with a blood vessel or other body lumen (not shown), and threaded through the tissue towards the vessel, e.g., by rotating the apparatus 1010 in a first direction. When the distal end 1022 of the plug member 1012 enters the vessel, fluid may flow into the distal opening 1028, through the lumens 1024, 1044 to a proximal outlet port or other bleed back indicator (not shown) on the handle device 1014. If desired, the apparatus 1 10 may be counter-rotated in a second direction until the plug member 1012 is disposed at a desired location within the passage.

The inner tube 1036 may then be advanced distally relative to the outer tube 1034, thereby engaging the sealing member 1032 and forcing it distally into the tapered portion 1025 of the lumen 1024. Because of its inherent flexibility and/or because of its coil shape, the sealing member 1025 may compress and/or otherwise become wedged into the tapered portion 1025, thereby substantially sealing the lumen 1024 from fluid flow therethrough, as shown in FIGS. 19C and 19D. The plug member 1012 may then be released from the handle device 1014, e.g., by withdrawing the outer tube 1036 (without rotating it to unthread the plug member 1012) and/or by releasing connectors securing the plug member 1012 to the distal end 1040 of the outer tube 1034. The handle device 1014 may then be withdrawn form the passage, leaving the plug member 1012 in place, similar to the embodiments described above.

Turning to FIGS. 20A-20F, an apparatus 1110 is shown that includes a plug member 1112 and an elongate shaft or handle device 1114. The plug member 1112 may be formed from biocompatible and/or bioabsorbable material, similar to the embodiments described above. The plug member 1112 includes a proximal end 1120, a distal end 1122, and an outer surface 1130, and a lumen 1124 that extends between the proximal and distal ends 1120, 1122. The plug member 1112 generally includes a helical thread pattern 1118, including one or more helical threads, that extend at least partially between its proximal and distal ends 1120, 1122. A sealing member 1132 may be provided within the lumen 1124, such as those described above.

The handle device 1114 has a proximal end 1134 and a distal end 1136, and defines a longitudinal axis 1138 that extends between the proximal and distal ends 1134, 1136. In one embodiment, the handle device 1114 is tubular, and includes a lumen 1140 extending between the proximal and distal ends 1134, 1136, the lumen 1140 communicating with the lumen 1124 when the plug member 1112 is attached to the distal end 1134 of the handle device 1114. A handle 1142 may be provided on the proximal end 1134 of the handle device 1114 for facilitating manipulation of the apparatus 1110, e.g., to facilitate rotation of the apparatus 1110 into a passage.

The handle device 1114 may be have a cross-section that is substantially smaller than a cross-section of the plug member 1112, e.g., to minimize dilation of a passage into which the apparatus 1110 is inserted. The plug member 1112 and/or the distal end 1136 of the handle device 1114 may include cooperating connectors (not shown) for releasably securing the plug member 1112 to the handle device 1114, as described above. Preferably, the handle 1142 includes an actuator (also not shown) that may be activated to release the connectors securing the plug member 1112 to the handle device 1114. Alternatively, the handle device 1114 may have a cross-section defining a portion of a circle, e.g., a "C" shape, or may include one or more elongate shafts (not shown) that releasably connect to the plug member 1112.

In addition, the plug member 1112 and the handle device 1114 include a bleed back device.for providing a visual indication when the distal end 1122 of the plug member 1112 is disposed within a blood vessel 90 or other body lumen. For example, as shown, the handle device 1114 may include a bleed back lumen 1152 that extends between the proximal and distal ends 1134, 1136. An outlet port 1145, a transparent tube (not shown) or other device may be provided on the proximal end 1134 of the handle device 1114 that communicates with the bleed back lumen 1152. The plug member 1112 may include a bleed back port 1154 that extends from the distal end 1122 to the bleed back lumen 1152. For example, the bleed back port 1154 may be a separate lumen (not shown) extending between the proximal and distal ends 1120, 1122 of the plug member 1112.

Alternatively, the bleed back port 1154 may be a groove extending along the lumen 1124 of the plug member 1112, as shown in FIGS. 20B-20E. In addition or alternatively, the bleed back lumen 1152 in the handle device 1114 may be a groove (not shown) extending along the lumen 1140 between the proximal and distal ends 1134, 1136 of the handle device 1114. The groove(s) may define a substantially enclosed passage when the apparatus 1110 is received over an introducer sheath 1116 or other elongate member.

The apparatus 1110 may be used in conjunction with an introducer sheath 1116 or other elongate member, having a proximal end 1144 and a distal end 1146. The sheath 1116 may include a tapered and/or substantially atraumatic distal end 1146 having a size for insertion through a puncture into a body lumen 90. The sheath 1116 may include a lumen 1150 that extends between the proximal and distal ends 1144, 1146, the lumen 1150 having a size to accommodate insertion of one or more devices therethrough. The sheath 1116 may include a seal (not shown), e.g., in the lumen 1150 adjacent the proximal end 1144 to substantially seal the lumen 1150, yet accommodate devices (not shown) therethrough.

The apparatus 1110 may be attachable to the sheath 1116 at any time during a procedure, e.g., such that the plug member 1112 and handle device 1114 may slide along the sheath 1116. Alternatively, the apparatus 1110 may be substantially permanently, but slidably, attached to the sheath 1116.

With particular reference to FIG. 20A, initially, the sheath 1116 may be introduced into a puncture or other passage 92 communicating with a blood vessel or other body lumen 90. The distal end 1146 may be introduced over a guidewire, a trocar, and/or a dilator (all not shown) until the distal end 1146 is disposed in or adjacent to the vessel 90, as is well known to those skilled in the art. One or more instruments or devices (not shown) may be advanced through the lumen 1150 into the vessel 90 to perform a procedure at a location within the patient's body accessed by the vessel 90. For example, an endovascular procedure may be performed within a patient's vasculature, such as angioplasty, stenting, atherectomy, and the like.

As shown in FIG. 20B, upon completing the procedure, any devices may be removed from the lumen 1150, and the plug member 1112 may be delivered into the passage 92 using the apparatus 1110. With the apparatus 1110 assembled, as shown, the apparatus 110 may be introduced into the passage 92 over the sheath 1116, i.e., by inserting the proximal end 1144 of the sheath 1116 into the lumen 1124 in the plug member 1112 and through the lumen 1140 in the handle device 1114.

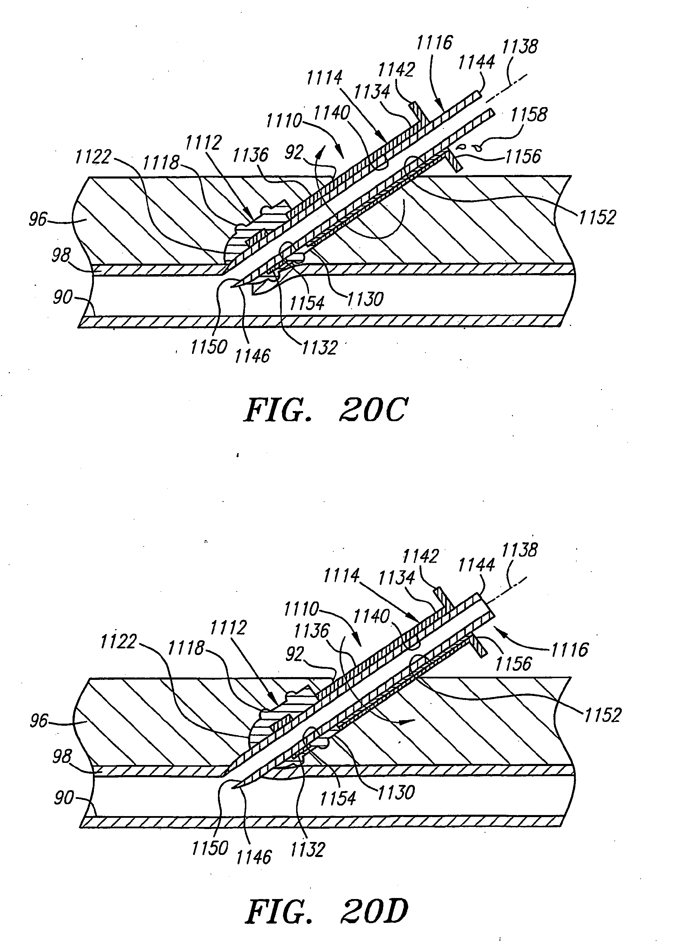

For example, the distal end 1122 of the plug member 1112 may be inserted into the passage 92 until the thread pattern 1118 begins to engage tissue 96 surrounding the passage 92. Then, as best seen in FIG. 20C, the handle device 1114 may be rotated in a first direction (indicated by exemplary arrow) to thread the plug member 1112 into the passage 92 over the sheath 1114. Consequently, the outer surface 1130 and/or thread pattern 1118 of the plug member 1112 may substantially engage tissue 96 surrounding the passage 92, thereby substantially sealing the passage 92 from fluids, such as blood, within the vessel 90.

Turning to FIG. 20C, as the plug member 1112 is advanced, the distal end 1122 of the plug member 1112 may eventually pass through the wall 98 of the vessel 90, whereupon the bleed back port 1154 may become exposed to fluid, i.e., blood, within the vessel 90. Because of internal blood pressure, the fluid enters the bleed back port 1154, passes through the bleed back lumen 1152, and exits the outlet port 1156 (as represented by drops 1158), thereby providing a visual indication that the vessel 90 has been reached.

As shown in FIG. 20D, the apparatus 1110 may be counter-rotated to withdraw the plug member 1112 away from the wall 98 of the vessel 90. For example, bleed back may occur when the plug member 1112 is within or in close proximity to the vessel 90, as shown in FIG. 20C. Rotation of the apparatus 1110 may then be reversed, i.e., in a second direction opposite the first direction (indicated by exemplary arrow), to withdraw the plug member 1112 a predetermined distance relative to the vessel 90, as shown in FIG. 20D. Because of the known pitch of the thread pattern 1118, the distance that the plug member 1112 is moved relative to the vessel 90 may be related directly to the number of rotationsand/or partial rotations that the apparatus 1110 is counter-rotated.

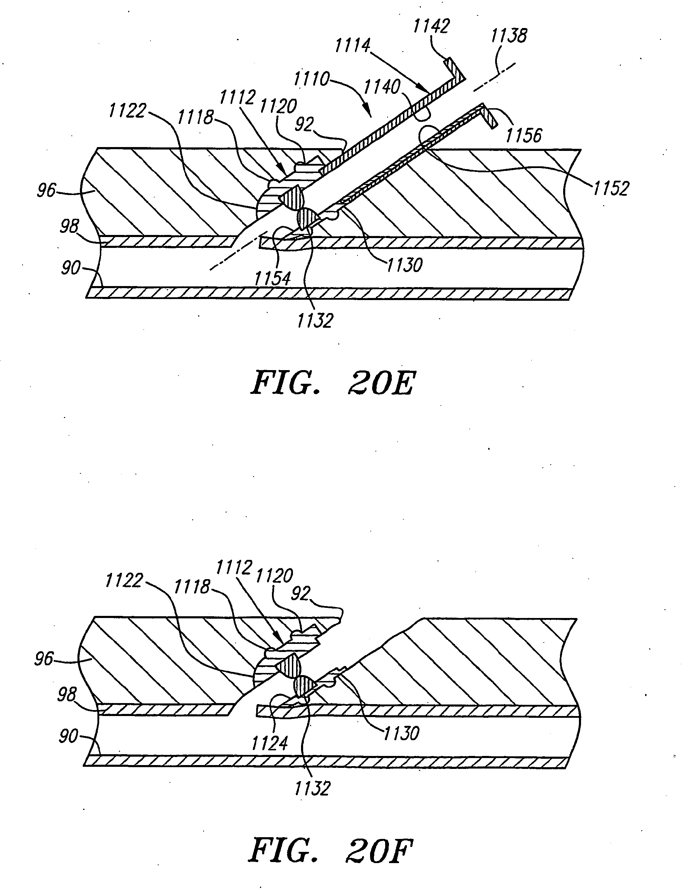

Once the desired deployment location is attained, the sheath 1116 may be removed, as shown in FIG. 20E. Alternatively, the sheath 1116 may be withdrawn earlier, e. g., when the distal end 1122 of the plug member 1112 is advanced into the vessel 90, or even earlier. As shown, the sealing member 1132 may expand to substantially seal the lumen 1124 in the plug member 1112, similar to the embodiments described above. For example; the sealing member 1132 may automatically expand when exposed to fluid, e. g., passing through the lumen 1124. Alternatively, the sealing member 1132 may be expanded to substantially seal the lumen 1124, either by an actuator or by compression of the sealing member 1132 (not shown), similar to the embodiments described above.

If desired, the bleed back port 1154, e. g., if a separate lumen (not shown) from the lumen 1124, may also include a sealing member (also not shown) for substantially sealing the bleed back port 1154 from fluid flow therethrough, similar to the sealing members described above.

When the plug member 12 is disposed at a desired location within the passage 92and/or once hemostasis is obtained, the plug member 1112 may be released from the handle device 1114. The handle device 1114 (and sheath 1116 if still within the passage 92) may be withdrawn from the passage 92. As shown in FIG. 20F, the plug member 1112 may remain in place to substantially seal the passage 92, e. g., until the plug member 1112 is absorbed by the patient's body. Alternatively, the plug member 1112 may be retrieved once the tissue between the plug member 1112 and the vessel 90 has substantially healed, as described above.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the scope of the appended claims.

## Claims

1. An apparatus (110) for sealing a passage through tissue, comprising:
an elongate shaft (112) having a proximal end (114) and a distal end (116);
an opening in the proximal end of the elongate shaft;
a plug member (120) disposed on the distal end (116) of the elongate shaft having a distal end (126), an opening in the distal end of the plug;
a position indicating lumen (136) therein which extends from the opening in the distal end of the plug to the opening in the proximal end of the elongate shaft;
wherein the elongate shaft (112) has a cross-section that is substantially smaller than a cross-section of the plug member (120).

2. The apparatus of claim 1, wherein the plug member (120) is at least partially tapered at its distal end.

3. The apparatus of claim 1, wherein the plug member (120) is substantially permanently attached to the distal end (116) of the elongate shaft (112).

4. The apparatus of claim 1, further comprising:
a lumen extending between the proximal and distal ends of said shaft; and wherein
the plug member (120) comprises a distal port therein in communication with the lumen.

5. The apparatus of claim 4, wherein the plug member (120) comprises a passage therein extending between the distal port and the lumen.

6. The apparatus (410) of claim 5, further comprising an elongate member (416) insertable through the lumen (440, 424) such that a distal end (446) of the elongate member is disposed beyond the distal end (422) of the plug member.

7. The apparatus of claim 6, wherein the distal end of the elongate member (416) comprises a location indicator for identifying when the distal end (422) of the plug member (420) is disposed adjacent a body lumen.

8. The apparatus of claim 7, wherein the elongate member (416) comprises a tubular member including a bleed back lumen (451), and wherein the location indicator comprises a bleed back port on the distal end (446) of the tubular member, the bleed back port being in communication with the bleed back lumen.

9. The apparatus of claim 7, wherein the location identifier comprises an expandable member, the expandable member being expandable when the distal end is disposed within a body lumen for providing tactile feedback of a location of the distal end of the plug member with respect to the body lumen.

10. The apparatus of claim 6, wherein the elongate member comprises an obturator (716) including a substantially atraumatic distal tip (746).

11. The apparatus of claim 6, further comprising a valve in the passage for substantially sealing the passage yet accommodating insertion of the elongate member therethrough.

12. The apparatus of claim 1 or claim 4, wherein the plug member (120) is releasable from the elongate shaft (112) or member.

13. The apparatus of claim 12, wherein the elongate shaft or member comprises an actuator (443) for releasing the plug (420) member from the distal end of the elongate shaft (414) or member.

14. The apparatus of claim 12, further comprising cooperating connectors on the distal end of the elongate shaft or member and on the plug member for releasably securing the plug member to the distal end of the elongate shaft or member.

15. The apparatus of claim 12, wherein the plug member comprises an interior cavity, and wherein the elongate shaft or member comprises an engagement element extending from the distal end thereof for insertion into the cavity, the engagement element being expandable and collapsible for engaging and disengaging an interior wall of the plug member, thereby selectively securing the plug member to and releasing the plug member from the distal end of the elongate shaft or member, respectively.

16. The apparatus of claim 15 when dependent upon claim 1, wherein the engagement element comprises a mechanically expandable frame.

17. The apparatus of claim 15 when dependent upon claim 1, wherein the engagement element comprises an inflatable member.

18. The apparatus of claim 15 when dependent upon claim 1, wherein the plug member is sufficiently rigid such that the plug member is substantially self-supporting when the engagement element is collapsed.

19. The apparatus of claim 1, further comprising:
an elongate member (416) comprising proximal (444) and distal (446) portions, and a groove (448) in an outer wall of the elongate member extending between the proximal and distal ends (444, 446);
a first lumen (440) extending between the proximal (434) and distal (430) ends of said shaft (414); and
the plug member (420) comprising a second lumen (424) extending therethrough that communicates with the first lumen (440),
wherein the elongate member (416) is slidable into the first and second lumens (440, 424) such that edges of the groove (448) substantially engage walls of the first and second lumens (440, 424) to define a bleed back lumen (451).

20. The apparatus of claim 19, wherein the plug member (420) comprises a helical thread on its outer surface.

21. The apparatus of claim 20, wherein the plug member (420) has a cross-section larger than a cross-section of the carrier member (414).

22. The apparatus of claim 5 or claim 19, further comprising a sealing member (432, 532, 1032) disposed in the passage or second lumen (424, 532, 1032) for substantially sealing the passage or second lumen (424, 532, 1032) from fluid flow therethrough.

23. The apparatus of claim 22, wherein the sealing member is biased towards a first configuration for substantially sealing the passage or second lumen from fluid flow therethrough, and is movable to a second configuration for accommodating introduction of one or more devices through the passage or second lumen.

24. The apparatus of claim 22, wherein the sealing member comprises a valve.

25. The apparatus of claim 22 when dependent upon claim 5, wherein the lumen (1024) includes a tapered portion (1025) reducing in cross-section, and wherein the sealing member (1032) comprises a generally annular-shaped member disposed adjacent a wide end of the tapered portion (1025) of the lumen (1024), the annular-shaped member being movable into the tapered portion (1025) for substantially sealing the lumen (1024).

26. The apparatus of claim 25, further comprising an activation element (1036) coupled to the elongate member, the activation element extending into the lumen (1024) of the plug member for moving the sealing member (1032) into the tapered portion (1025) for substantially sealing the lumen.

27. The apparatus of claim 22 when dependent upon claim 19, wherein the sealing member (532, 1032) comprises a generally annular-shaped member disposed within the second lumen (524, 1024), the apparatus further comprising an activation element (536, 1036) coupled to the carrier member, the activation element being movable within the second lumen (524, 1024) for deforming the annular-shaped member to substantially seal the second lumen.

28. The apparatus of claim 27, wherein the second lumen comprises a tapered portion (1025) reducing in cross-section, and wherein the annular-shaped member (1032) is movable into the tapered portion (1035) by the activation element (1036) for substantially sealing the second lumen.

29. The apparatus of claim 27, wherein a distal end of the annular-shaped member (532) is secured from distal movement, and wherein the activation element (536) is configured for compressing a proximal end of the annular-shaped member towards the distal end thereof to wedge the annular-shaped member (532) into the second lumen (524).

30. The apparatus of claim 19, wherein the elongate member comprises a tubular member.

31. The apparatus of claim 30, wherein the tubular member comprises a guidewire lumen (456) extending between the proximal and distal ends for receiving a guidewire therethrough.

32. The apparatus of claim 19, wherein the elongate member comprises a plurality of grooves extending between the proximal and distal portions.

33. The apparatus of claim 19, wherein the elongated shaft comprises a carrier member and the plug member (420) is releasable from the carrier member (414).

34. The apparatus of claim 33, wherein the carrier member (414) comprises an actuator (443) for releasing the plug member (420) from the distal end (436) of the carrier member (414).

35. The apparatus of claim 34, further comprising cooperating connectors on the distal end (436) of the carrier member (414) and on the plug member (420) for releasably securing the plug member to the distal end of the carrier member.

36. The apparatus of claim 33, wherein the plug member comprises an interior cavity, and wherein the carrier member comprises an engagement element extending from the distal end thereof for insertion into the cavity, the engagement element being expandable and collapsible for engaging and disengaging an interior wall of the plug member, thereby selectively securing the plug member to and releasing the plug member from the distal end of the carrier member, respectively.

37. The apparatus of claim 12 or claim 33, wherein the plug member (120, 420) comprises bioabsorbable material.

38. The apparatus of claim 33, wherein rotation of the plug member is coupled to rotation of the carrier member until the plug member is released from the carrier member.

39. The apparatus of claim 1, wherein said plug member (120, 420) is rigidly attached to the distal end of the elongate shaft.

40. The apparatus of claim 1 or claim 39, wherein the plug member (420) comprises a cavity in its distal end.

41. The apparatus of claim 40, further comprising at least one of a hemostasis-promoting material and an infection-resistant material disposed in the cavity.

42. The apparatus of claim 41 when dependent upon claim 1, wherein the material comprises intestinal submucosa.

43. The apparatus of claim 1 or claim 39, further comprising at least one of a hemostasis-promoting material and an infection-resistant material secured to a distal end of the plug member (422).

44. The apparatus of claim 1 or claim 39, further comprising a lumen extending from the proximal end (114, 434) of the elongate shaft through the plug member (120, 420), and a seal (138, 432) for selectively sealing the lumen.

45. The apparatus of claim 44 when dependent upon claim 1 wherein the seal (138) is disposed on the proximal end (114) of the elongate shaft (112).

46. The apparatus of claim 1, wherein said plug member is formed from a bioabsorbable material, the plug member comprising a body having a proximal end and a substantially closed distal end.

47. The apparatus of claim 46, further comprising a connector on the proximal end for detachably securing the plug member to a delivery apparatus.

48. The apparatus of claim 46, wherein the body further comprises an internal cavity communicating with an opening in the proximal end.

49. The apparatus of claim 46, further comprising:
a lumen extending between the proximal end and a distal inlet port; and
a sealing member disposed within the lumen that is expandable across the lumen for substantially sealing the lumen from fluid flow therethrough.

50. The apparatus of claim 49, wherein the sealing member comprises an annular-shaped member.

51. The apparatus of claim 1, wherein said plug member (1020) forms a bioabsorbable body comprising a proximal end (1020), a distal end (1022), said body comprising a lumen (1024) extending between a proximal port and a distal port, the lumen comprising a tapered portion (1025) that tapers in cross-section; and
a sealing member (1032) comprising a generally annular-shaped member disposed adjacent a wide end of the tapered portion (1025) of the lumen (1024), the sealing member (1032) being movable into the tapered portion (1025) for substantially sealing the lumen (1024) from fluid flow therethrough.

52. The apparatus of any of claims 22, 49 and 51, wherein the sealing member (432, 532, 1032) comprises a material that is expandable when exposed to fluid.

53. The apparatus of claim 51, wherein the sealing member (1025) comprises a flexible material that may be wedged into the tapered portion.

54. The apparatus of claim 49 or claim 51, wherein the sealing member comprises a bioabsorbable material,

55. The apparatus of claim 51, further comprising a connector on the proximal end of the body for detachably securing the body to a delivery device.

56. The apparatus of claim 49 or claim 51, further comprising an elongate shaft extending from the proximal end of the body.

## Patentansprüche

1. Vorrichtung (110) zum Abdichten eines Durchlasses durch ein Gewebe, umfassend:
einen länglichen Schaft (112) mit einem proximalen Ende (114) und einem distalen Ende (116);
eine Öffnung in dem proximalen Ende des länglichen Schafts;
ein an dem distalen Ende (116) des länglichen Schafts angeordnetes Verschlusselement (120) mit einem distalen Ende (126) und einer Öffnung in dem distalen Ende des Verschlusselements;
ein Positions-anzeigendes Lumen (136) darin, welches sich von der Öffnung in dem distalen Ende des Verschlusselements zu der Öffnung in dem proximalen Ende des länglichen Schafts erstreckt;
wobei der längliche Schaft (112) einen Querschnitt aufweist, welcher wesentlich kleiner ist als ein Querschnitt des Verschlusselements (120).

2. Vorrichtung nach Anspruch 1, wobei sich das Verschlusselement (120) an seinem distalen Ende wenigstens teilweise verjüngt.

3. Vorrichtung nach Anspruch 1, wobei das Verschlusselement (120) im wesentlichen dauerhaft an dem distalen Ende (116) des länglichen Schafts (112) befestigt ist.

4. Vorrichtung nach Anspruch 1, ferner umfassend:
ein sich zwischen dem proximalen und dem distalen Ende des Schafts erstreckendes Lumen; und wobei
das Verschlusselement (120) eine distale Öffnung darin umfasst, die in Verbindung zu dem Lumen steht.

5. Vorrichtung nach Anspruch 4, wobei das Verschlusselement (120) einen Durchlass darin umfasst, welcher sich zwischen der distalen Öffnung und dem Lumen erstreckt.

6. Vorrichtung (410) nach Anspruch 5, welche ferner ein durch das Lumen (440, 424) einführbares längliches Element (416) umfasst, so dass ein distales Ende (446) des länglichen Elements hinter dem distalen Ende (422) des Verschlusselements angeordnet ist.

7. Vorrichtung nach Anspruch 6, wobei das distale Ende des länglichen Elements (416) einen Ortsanzeiger umfasst, um zu erfassen, dass das distale Ende (422) des Verschlusselements (420) angrenzend an ein Körperlumen angeordnet ist.

8. Vorrichtung nach Anspruch 7, wobei das längliche Element (416) ein röhrenförmiges Element umfasst, welches ein Rückblutungslumen (451) umfasst, und wobei der Ortsanzeiger eine Rückblutungsöffnung an dem distalen Ende (446) des röhrenförmigen Elements umfasst, wobei die Rückblutungsöffnung in Verbindung zu dem Rückblutungslumen steht.

9. Vorrichtung nach Anspruch 7, wobei der Ortserfasser ein expandierbares Element umfasst, wobei das expandierbare Element expandierbar ist, wenn das distale Ende in einem Körperlumen angeordnet ist, um eine taktile Rückmeldung einer Position des distalen Endes des Verschlusselements bezüglich des Körperlumens zu geben.

10. Vorrichtung nach Anspruch 6, wobei das längliche Element ein Verschlussstück (716), umfassend eine im Wesentlichen atraumatische distale Spitze (746), umfasst.

11. Vorrichtung nach Anspruch 6, welche ferner ein Ventil in dem Durchlass umfasst, um den Durchlass im Wesentlichen abzudichten, aber das Einführen des länglichen Elements dort hindurch zu erlauben.

12. Vorrichtung nach Anspruch 1 oder Anspruch 4, wobei das Verschlusselement (120) von dem länglichen Schaft (112) oder Element lösbar ist.

13. Vorrichtung nach Anspruch 12, wobei der längliche Schaft oder das längliche Element ein Bedienteil (443) zum Freigeben des Verschlusselements (420) von dem distalen Ende des länglichen Schafts oder Elements (414) umfasst.

14. Vorrichtung nach Anspruch 12, welche ferner zusammen wirkende Verbindungselemente an dem distalen Ende des länglichen Schafts oder Elements und an dem Verschlusselement umfasst, um das Verschlusselement lösbar an dem distalen Ende des länglichen Schafts oder Elements zu befestigen.

15. Vorrichtung nach Anspruch 12, wobei das Verschlusselement einen inneren Hohlraum umfasst, und wobei der längliche Schaft oder das längliche Element ein Eingreifelement umfasst, welches sich zum Einführen in den Hohlraum von dem distalen Ende daran erstreckt, wobei das Eingreifelement expandierbar und kollabierbar ist, um eine Innenwand des Verschlusselements einzugreifen und freizugeben, wodurch das Verschlusselement selektiv an dem distalen Ende des länglichen Schafts oder Elements befestigt beziehungsweise von ihm freigegeben wird.

16. Vorrichtung nach Anspruch 15, wenn von Anspruch 1 abhängig, wobei das Eingreifelement einen mechanisch expandierbaren Rahmen umfasst.

17. Vorrichtung nach Anspruch 15, wenn von Anspruch 1 abhängig, wobei das Eingreifelement ein aufblasbares Element umfasst.

18. Vorrichtung nach Anspruch 15, wenn von Anspruch 1 abhängig, wobei das Verschlusselement ausreichend steif ist, so dass das Verschlusselement im Wesentlichen selbsttragend ist, wenn das Eingreifelement kollabiert ist.

19. Vorrichtung nach Anspruch 1, ferner umfassend:
ein längliches Element (416), welches einen proximalen (444) und einen distalen (446) Abschnitt umfasst, sowie eine Nut (448) an einer Außenwand des länglichen Elements, die sich zwischen dem proximalen und dem distalen Ende (444, 446) erstreckt;
ein sich zwischen dem proximalen (434) und dem distalen (430) Ende des Schafts erstreckendes erstes Lumen (440); und
das Verschlusselement (420), welches ein sich hindurch erstreckendes zweites Lumen (424) umfasst, das in Verbindung mit dem ersten Lumen (440) steht,
wobei das längliche Element (416) in das erste und das zweite Lumen (440, 424) verschiebbar ist, so dass die Ränder der Nut (448) im Wesentlichen in Wände des ersten und des zweiten Lumens (440, 424) eingreifen, um ein Rückblutungslumen (451) zu definieren.

20. Vorrichtung nach Anspruch 19, wobei das Verschlusselement (420) an seiner Außenfläche ein schraubenförmiges Gewinde umfasst.

21. Vorrichtung nach Anspruch 20, wobei das Verschlusselement (420) einen Querschnitt aufweist, welcher größer ist als ein Querschnitt des Trägerelements (414).

22. Vorrichtung nach Anspruch 5 oder Anspruch,19, welche ferner ein Abdichtungselement (432, 532, 1032) umfasst, das in dem Durchlass oder zweiten Lumen (424, 532, 1032) angeordnet ist, um im Wesentlichen den Durchlass oder das zweite Lumen (424, 532, 1032) gegen einen Fluidfluss dort hindurch abzudichten.

23. Vorrichtung nach Anspruch 22, wobei das Abdichtungselement zu einer ersten Konfiguration vorgespannt ist, um den Durchlass oder das zweite Lumen im Wesentlichen gegen einen Fluidfluss dort hindurch abzudichten, und zu einer zweiten Konfiguration bewegbar ist, um die Einführung einer oder mehrere Vorrichtungen durch den Durchlass oder das zweite Lumen zu ermöglichen.

24. Vorrichtung nach Anspruch 22, wobei das Abdichtungselement ein Ventil umfasst.

25. Vorrichtung nach Anspruch 22, wenn von Anspruch 5 abhängig, wobei das Lumen (1024) einen sich verjüngenden Abschnitt (1025) umfasst, der sich im Querschnitt verringert, und wobei das Abdichtungselement (1032) ein im Allgemeinen ringförmiges Element umfasst, das neben einem breiten Ende des sich verjüngenden Abschnitts (1025) des Lumens (1024) angeordnet ist, wobei das ringförmige Element in den sich verjüngenden Abschnitt (1025) bewegbar ist, im das Lumen (1024) im Wesentlichen abzudichten.

26. Vorrichtung nach Anspruch 25, welche ferner ein an das längliche Element gekoppeltes Betätigungselement (1036) umfasst, wobei sich das Betätigungselement in das Lumen (1024) des Verschlusselements erstreckt, um das Abdichtungselement (1032) in den sich verjüngenden Abschnitt (1025) zu bewegen, um das Lumen im Wesentlichen abzudichten.

27. Vorrichtung nach Anspruch 22, wenn von Anspruch 19 abhängig, wobei das Abdichtungselement (532, 1032) ein in dem zweiten Lumen (524, 1024) angeordnetes, im Wesentlichen ringförmiges Element umfasst, wobei die Vorrichtung ferner ein an das Trägerelement gekoppeltes Betätigungselement (536, 1036) umfasst, wobei das Betätigungselement in dem zweiten Lumen (524, 1024) bewegbar ist, um das ringförmige Element zu verformen, um das zweite Lumen im Wesentlichen abzudichten.

28. Vorrichtung nach Anspruch 27, wobei das zweite Lumen einen sich verjüngenden Abschnitt (1025) mit sich verringerndem Querschnitt umfasst, und wobei das ringförmige Element (1032) durch das Betätigungselement (1036) in den sich verjüngenden Abschnitt (1035) bewegbar ist, um das zweite Lumen im Wesentlichen abzudichten.

29. Vorrichtung nach Anspruch 27, wobei ein distales Ende des ringförmigen Elements (532) gegen distale Bewegung gesichert ist, und wobei das Betätigungselement (536) für das Komprimieren eines proximalen Endes des ringförmigen Elements zu dessen distalen Ende hin ausgebildet ist, um das ringförmige Element (532) in das zweite Lumen (524) zu zwängen.

30. Vorrichtung nach Anspruch 19, wobei das längliche Element ein röhrenförmiges Element umfasst.

31. Vorrichtung nach Anspruch 30, wobei das röhrenförmige Element ein Führungsdrahtlumen (456) umfasst, welches sich zwischen dem proximalen und dem distalen Ende erstreckt, um einen Führungsdraht in sich aufzunehmen.

32. Vorrichtung nach Anspruch 19, wobei das längliche Element eine Mehrzahl von Nuten umfasst, die sich zwischen dem proximalen und dem distalen Abschnitt erstrecken.

33. Vorrichtung nach Anspruch 19, wobei der längliche Schaft ein Trägerelement umfasst und wobei das Verschlusselement (420) von dem Trägerelement (414) lösbar ist.

34. Vorrichtung nach Anspruch 33, wobei das Trägerelement (414) ein Betätigungselement (443) umfasst, um das Verschlusselement (420) von dem distalen Ende (436) des Trägerelements (414) zu lösen.

35. Vorrichtung nach Anspruch 34, welche ferner zusammen wirkende Verbindungselemente an dem distalen Ende (436) des Trägerelements (414) und an dem Verschlusselement (420) umfasst, um das Verschlusselement lösbar an dem distalen Ende des Trägerelements zu befestigen.

36. Vorrichtung nach Anspruch 33, wobei das Verschlusselement einen inneren Hohlraum umfasst, und wobei das Trägerelement ein Eingreifelement umfasst, das sich von dessen distalen Ende für die Einführung in den Hohlraum erstreckt, wobei das Eingreifelement expandierbar und kollabierbar ist, um eine Innenwand des Verschlusselements einzugreifen und freizugeben, wodurch das Verschlusselement selektiv an dem distalen Ende des Trägerelements befestigt beziehungsweise von ihm freigegeben wird.

37. Vorrichtung nach Anspruch 12 oder Anspruch 33, wobei das Verschlusselement (120, 420) ein bioabsorbierbares Material umfasst.

38. Vorrichtung nach Anspruch 33, wobei die Drehung des Verschlusselements an die Drehung des Trägerelements gekoppelt ist bis das Verschlusselement von dem Trägerelement freigegeben wird.

39. Vorrichtung nach Anspruch 1, wobei das Verschlusselement (120, 420) fest an dem distalen Ende des länglichen Schafts befestigt ist.

40. Vorrichtung nach Anspruch 1 oder Anspruch 39, wobei das Verschlusselement (420) einen Hohlraum in seinem distalen Ende umfasst.

41. Vorrichtung nach Anspruch 40, welche ferner wenigstens eins eines Hämostase-fördernden Materials oder eines Infektions-resistenten Materials in dem Hohlraum angeordnet umfasst.

42. Vorrichtung nach Anspruch 41, wenn abhängig von Anspruch 1, wobei das Material intestinale Submukosa umfasst.

43. Vorrichtung nach Anspruch 1 oder Anspruch 39, welche ferner wenigstens eins eines Hämostase-fördernden Materials oder eines Infektions-resistenten Materials an einem distalen Ende des Verschlusselements (422) befestigt umfasst.

44. Vorrichtung nach Anspruch 1 oder Anspruch 39, welche ferner ein Lumen, welches sich von dem proximalen Ende (114, 434) des länglichen Schafts durch das Abschlusselement (120, 420) erstreckt, und eine Abdichtung (138, 432) zum selektiven Abdichten des Lumens umfasst.

45. Vorrichtung nach Anspruch 44, wenn von Anspruch 1 abhängig, wobei die Abdichtung (138) am proximalen Ende (114) des länglichen Schafts (112) angeordnet ist.

46. Vorrichtung nach Anspruch 1, wobei das Abschlusselement aus einem bioabsorbierbaren Materal gebildet ist, und wobei das Abschlusselement einen Körper mit einem proximalen Ende und einem im Wesentlichen geschlossenen distalen Ende umfasst.

47. Vorrichtung nach Anspruch 46, welche ferner ein Verbindungselement am proximalen Ende umfasst, um das Abschlusselement lösbar an einer Einbringungsvorrichtung zu befestigen.

48. Vorrichtung nach Anspruch 46, wobei der Körper ferner einen inneren Hohlraum umfasst, der in Verbindung zu einer Öffnung am proximalen Ende steht.

49. Vorrichtung nach Anspruch 46, ferner umfassend:
ein sich zwischen dem proximalen Ende und einer distalen Einlassöffnung erstreckendes Lumen; und
ein in dem Lumen angeordnetes Abdichtungselement, welches durch das Lumen expandierbar ist, um das Lumen im Wesentlichen gegen einen Fluidfluss dort hindurch abzudichten.

50. Vorrichtung nach Anspruch 49, wobei das Abdichtungselement ein ringförmiges Element umfasst.

51. Vorrichtung nach Anspruch 1, wobei das Abschlusselement (1020) einen bioabsorbierbaren Körper bildet, umfassend ein proximales Ende (1020), ein distales Ende (1022), wobei der Körper ein sich zwischen einer proximalen Öffnung und einer distalen Öffnung erstreckendes Lumen (1024) umfasst,
wobei das Lumen einen sich verjüngende Abschnitt (1025) umfasst, der sich im Querschnitt verjüngt; und
ein Abdichtungselement (1032), umfassend ein neben einem breiten Ende des sich verjüngenden Abschnitts (1025) des Lumens (1024) angeordnetes, im Allgemeinen ringförmiges Element, wobei das Abdichtungselement (1032) in den sich verjüngenden Abschnitt (1025) bewegbar ist, um das Lumen (1024) im Wesentlichen gegen einen Fluidfluss dort hindurch abzudichten.

52. Vorrichtung nach einem der Ansprüche 22, 49 und 51, wobei das Abdichtungselement (432, 532, 1032) ein Material umfasst, welches expandierbar ist, wenn es Fluid ausgesetzt ist.

53. Vorrichtung nach Anspruch 51, wobei das Abdichtungselement (1025) ein flexibles Material umfasst, welches in den sich verjüngenden Abschnitt gedrückt werden kann.

54. Vorrichtung nach Anspruch 49 oder Anspruch 51, wobei das Abdichtungselement ein bioabsorbierbares Material umfasst.

55. Vorrichtung nach Anspruch 51, welche ferner zum lösbaren Befestigen des Körpers an einer Einbringungsvorrichtung ein Verbindungselement am proximalen Ende des Körpers umfasst.

56. Vorrichtung nach Anspruch 49 oder Anspruch 51, welche ferner einen länglichen Schaft umfasst, der sich vom proximalen Ende des Körpers erstreckt.

## Revendications

1. Appareil (110) pour obturer un passage dans un tissu, comportant :
une tige allongée (112) ayant une extrémité proximale (114) et une extrémité distale (116) ;
une ouverture dans l'extrémité proximale de la tige allongée ;
un organe formant bouchon (120) disposé sur l'extrémité distale (116) de la tige allongée ayant une extrémité distale (126), une ouverture dans l'extrémité distale de l'obturateur ;
une lumière (136) d'indication de position dans celui-ci, qui s'étend de l'ouverture située dans l'extrémité distale de l'obturateur jusqu'à l'ouverture située dans l'extrémité proximale de la tige allongée ;
dans lequel la tige allongée (112) a une section transversale qui est sensiblement inférieure à la section transversale de l'élément obturateur (120).

2. Appareil selon la revendication 1, dans lequel l'élément obturateur (120) est au moins partiellement effilé à son extrémité distale.

3. Appareil selon la revendication 1, dans lequel l'organe obturateur (120) est attaché de façon sensiblement permanente à l'extrémité distale (116) de la tige allongée (112).

4. Appareil selon la revendication 1, comportant en outre :
une lumière s'étendant entre les extrémités proximale et distale de ladite tige ; et dans lequel
l'organe obturateur (120) présente en lui un orifice distal en communication avec la lumière.

5. Appareil selon la revendication 4, dans lequel l'organe obturateur (120) renferme un passage s'étendant entre l'orifice distal et la lumière.

6. Appareil (410) selon la revendication 5, comportant en outre un organe allongé (416) pouvant être inséré dans la lumière (440, 424) de manière qu'une extrémité distale (446) de l'organe allongé soit disposée au-delà de l'extrémité distale (422) de l'organe formant bouchon.

7. Appareil selon la revendication 6, dans lequel l'extrémité distale de l'organe allongé (416) comporte un indicateur de position destiné à identifier le moment où l'extrémité distale (422) de l'organe formant bouchon (420) est disposée de façon à être adjacente à une lumière corporelle.

8. Appareil selon la revendication 7, dans lequel l'organe allongé (416) comprend un organe tubulaire ayant une lumière (451) de reflux, et dans lequel l'indicateur de position comprend un orifice de reflux sur l'extrémité distale (446) de l'élément tubulaire, l'orifice de reflux étant en commutation avec la lumière de reflux.

9. Appareil selon la revendication 7, dans lequel l'identificateur de position comprend un organe expansible, l'organe expansible pouvant être expansé lorsque l'extrémité distale est disposée à l'intérieur d'une lumière corporelle pour fournir un retour tactile d'un emplacement de l'extrémité distale de l'organe formant bouchon par rapport à la lumière corporelle.

10. Appareil selon la revendication 6, dans lequel l'organe allongé comporte un obturateur (716) comprenant un bout distal sensiblement atraumatique (746).

11. Appareil selon la revendication 6, comportant en outre une valve dans le passage pour obturer sensiblement le passage tout en permettant l'insertion de l'organe allongé dans ce passage.

12. Appareil selon la revendication 1 ou la revendication 4, dans lequel l'organe formant bouchon (120) peut être libéré de la tige (112) ou organe allongé.

13. Appareil selon la revendication 12, dans lequel la tige ou l'organe allongé comprend un actionneur (443) pour libérer l'organe formant bouchon (420) de l'extrémité distale de la tige (414) ou organe allongé.

14. Appareil selon la revendication 12, comportant en outre des raccords coopérant sur l'extrémité distale de la tige ou organe allongé et sur l'organe formant bouchon pour fixer de façon libérable l'organe formant bouchon à l'extrémité distale de la tige ou organe allongé.

15. Appareil selon la revendication 12, dans lequel l'organe formant bouchon présente une cavité intérieure, et dans lequel la tige ou organe allongé comporte un élément d'engagement s'étendant depuis son extrémité distale pour être inséré dans la cavité, l'élément d'engagement étant expansible et rétractable pour engager une paroi intérieure de l'organe formant bouchon et s'en dégager, de manière ainsi à sélectivement fixer l'organe formant bouchon à l'extrémité distale de la tige ou organe allongé et libérer l'organe formant bouchon de l'extrémité distale de la tige ou organe allongé, respectivement.

16. Appareil selon la revendication 15, lorsqu'elle dépend de la revendication 1, dans lequel l'élément d'engagement comprend un cadre mécaniquement expansible.

17. Appareil selon la revendication 15, lorsqu'elle dépend de la revendication 1, dans lequel l'élément d'engagement comprend un organe gonflable.

18. Appareil selon la revendication 15, lorsqu'elle dépend de la revendication 1, dans lequel l'organe formant bouchon est suffisamment rigide pour que l'organe formant bouchon soit sensiblement autoportant lorsque l'élément d'engagement est rétracté.

19. Appareil selon la revendication 1, comportant en outre :
un organe allongé (416) comportant des parties proximale (444) et distale (446), et une gorge (448) dans une paroi extérieure de l'organe allongé s'étendant entre les extrémités proximale et distale (444, 446) ;
une première lumière (440) s'étendant entre les extrémités proximale (443) et distale (430) de ladite tige (414) ; et
l'organe formant bouchon (420) comportant une seconde lumière (424) s'étendant à travers lui qui communique avec la première lumière (440),
dans lequel l'organe allongé (416) peut être introduit en coulissant dans les première et seconde lumières (440, 424) de manière que des bords de la gorge (448) engagent sensiblement des parois des première et seconde lumières (440, 424) pour définir une lumière de reflux (451).

20. Appareil selon la revendication 19, dans lequel l'organe formant bouchon (420) comporte un cliquetage hélicoïdal sur sa surface extérieure.

21. Appareil selon la revendication 20, dans lequel l'organe formant bouchon (420) a une section transversale plus grande qu'une section transversale de l'organe de support (414).

22. Appareil selon la revendication 5 ou la revendication 19, comportant en outre un organe d'obturation (432, 532, 1032) disposé dans le passage ou dans la seconde lumière (424, 532, 1032) pour obturer sensiblement le passage ou la seconde lumière (424, 532, 1032) afin d'empêcher tout fluide de s'y écouler.

23. Appareil selon la revendication 22, dans lequel l'organe d'obturation est rappelé vers une première configuration pour obturer sensiblement le passage ou la seconde lumière afin d'empêcher tout fluide de s'y écouler, et peut être amené dans une seconde configuration pour permettre l'introduction d'un ou plusieurs dispositifs à travers le passage ou la seconde lumière.

24. Appareil selon la revendication 22, dans lequel l'organe d'obturation comprend une valve.

25. Appareil selon la revendication 22 lorsqu'elle dépend de la revendication 5, dans lequel la lumière (1024) comprend une partie effilée (1025) dont la section transversale diminue, et dans lequel l'organe d'obturation (1032) comprend un organe de forme globalement annulaire disposé de façon à être adjacent à une extrémité large de la partie effilée (1025) de la lumière (1024), l'organe de forme annulaire pouvant être introduit dans la partie effilée (1025) pour obturer sensiblement la lumière (1024).

26. Appareil selon la revendication 25, comportant en outre un élément d'activation (1036) relié à l'organe allongé, l'élément d'activation s'étendant dans la lumière (1024) de l'organe formant bouchon pour amener l'organe d'obturation (1032) dans la partie effilée (1025) afin d'obturer sensiblement la lumière.

27. Appareil selon la revendication 22 lorsqu'elle dépend de la revendication 19, dans lequel l'organe d'obturation (532, 1032) comprend un organe de forme globalement annulaire disposé à l'intérieur de la seconde lumière (524, 1024), l'appareil comportant en outre un élément d'activation (536, 1036) relié à l'organe de support, l'élément d'activation pouvant être introduit dans la seconde lumière (524, 1024) pour déformer l'organe de forme annulaire afin d'obturer sensiblement la seconde lumière.

28. Appareil selon la revendication 27, dans lequel la seconde lumière comprend une partie effilée (1025) dont la section transversale diminue, et dans lequel l'organe de forme annulaire (1032) peut être introduit dans la partie effilée (1035) par l'élément d'activation (1036) pour obturer sensiblement la seconde lumière.

29. Appareil selon la revendication 27, dans lequel une extrémité distale de l'organe (532) de forme annulaire est fixée de façon à ne pas se déplacer du côté distal, et dans lequel l'élément d'activation (536) est configuré pour comprimer une extrémité proximale de l'organe de forme annulaire vers son extrémité distale afin de coincer l'organe (532) de forme annulaire dans la seconde lumière (524).

30. Appareil selon la revendication 19, dans lequel l'organe allongé comprend un organe tubulaire.

31. Appareil selon la revendication 30, dans lequel l'organe tubulaire comprend une lumière (456) pour fil de guidage s'étendant entre les extrémités proximale et distale pour recevoir à travers elle un fil de guidage.

32. Appareil selon la revendication 19, dans lequel l'organe allongé comporte de multiples gorges s'étendant entre les parties proximale et distale.

33. Appareil selon la revendication 19, dans lequel la tige allongée comporte un organe de support et l'organe formant bouchon (420) peut être libéré de l'organe de support (414).

34. Appareil selon la revendication 33, dans lequel l'organe de support (414) comporte un actionneur (443) pour libérer l'organe formant bouchon (420) de l'extrémité distale (436) de l'organe de support (414).

35. Appareil selon la revendication 34, comportant en outre des raccords coopérant sur l'extrémité distale (436) de l'organe de support (414) et sur l'organe formant bouchon (420) pour fixer de façon libérable l'organe formant bouchon et l'extrémité distale de l'organe de support.

36. Appareil selon la revendication 33, dans lequel l'organe formant bouchon présente une cavité intérieure, et dans lequel l'organe de support comporte un élément d'engagement s'étendant depuis son extrémité distale pour être inséré dans la cavité, l'élément d'engagement étant expansible et rétractable pour engager une paroi intérieure de l'organe formant bouchon et s'en dégager, de manière, ainsi, à sélectivement fixer l'organe formant bouchon à l'extrémité distale de l'organe de support et libérer l'organe formant bouchon de l'extrémité distale de l'organe de support, respectivement.

37. Appareil selon la revendication 12 ou la revendication 33, dans lequel l'organe formant bouchon (120, 420) comprend une matière bio-absorbable.

38. Appareil selon la revendication 33, dans lequel une rotation de l'organe formant bouchon est couplée à une rotation de l'organe de support jusqu'à ce que l'organe formant bouchon soit libéré de l'organe de support.

39. Appareil selon la revendication 1, dans lequel ledit organe formant bouchon (120, 420) est attaché rigidement à l'extrémité distale de la tige allongée.

40. Appareil selon la revendication 1 ou la revendication 39, dans lequel l'organe formant bouchon (420) présente une cavité dans son extrémité distale.

41. Appareil selon la revendication 40, comportant en outre au moins l'une d'une matière favorisant l'hémostase et d'une matière résistant à l'infection, disposée dans la cavité.

42. Appareil selon la revendication 41 lorsqu'elle dépend de la revendication 1, dans lequel la matière comprend une sous-muqueuse intestinale.

43. Appareil selon la revendication 1 ou la revendication 39, comportant en outre au moins l'une d'une matière favorisant l'hémostase et d'une matière résistant à l'infection fixée à une extrémité distale de l'organe formant bouchon (422).

44. Appareil selon la revendication 1 ou la revendication 39, comportant en outre une lumière s'étendant depuis l'extrémité proximale (114, 434) de la tige allongée à travers l'organe formant bouchon (120, 420), et un élément d'obturation (138, 432) destiné à obturer sélectivement la lumière.

45. Appareil selon la revendication 44 lorsqu'elle dépend de la revendication 1, dans lequel l'élément d'obturation (138) est disposé sur l'extrémité proximale (114) de la tige allongée (112).

46. Appareil selon la revendication 1, dans lequel ledit organe formant bouchon est formé d'une matière bio-absorbable, l'organe formant bouchon comprenant un corps ayant une extrémité proximale et une extrémité distale sensiblement fermée.

47. Appareil selon la revendication 46, comportant en outre un raccord sur l'extrémité proximale pour relier de façon séparable l'organe formant bouchon à un appareil de distribution.

48. Appareil selon la revendication 46, dans lequel le corps présente en outre une cavité intérieure communiquant avec une ouverture dans l'extrémité proximale.

49. Appareil selon la revendication 46, comportant en outre :
une lumière s'étendant entre l'extrémité proximale et un orifice d'entrée distal ; et
un organe d'obturation disposé à l'intérieur de la lumière, qui peut être expansé en travers de la lumière pour obturer sensiblement la lumière afin d'empêcher tout fluide de s'y écouler.

50. Appareil selon la revendication 49, dans lequel l'organe d'obturation comprend un organe de forme annulaire.

51. Appareil selon la revendication 1, dans lequel ledit organe formant bouchon (1020) forme un corps bio-absorbable ayant une extrémité proximale (1020), une extrémité distale (1022), ledit corps présentant une lumière (1024) s'étendant entre un orifice proximal et un orifice distal, la lumière comprend une partie effilée (1025) qui diminue en section transversale ; et
un organe d'obturation (1032) comprend un organe de forme globalement annulaire disposé de façon à être adjacent à une extrémité large de la partie effilée (1025) de la lumière (1024), l'organe d'obturation (1032) pouvant être introduit dans la partie effilée (1025) pour obturer sensiblement la lumière (1024) afin d'empêcher tout fluide de s'y écouler.

52. Appareil selon l'une quelconque des revendications 22, 49 et 51, dans lequel l'organe d'obturation (432, 532, 1032) comprend une matière qui est expansible lorsqu'elle est exposée à un fluide.

53. Appareil selon la revendication 51, dans lequel l'organe d'obturation (1025) comprend une matière flexible qui peut être coincée dans la partie effilée.

54. Appareil selon la revendication 49, ou la revendication 51, dans lequel l'organe d'obturation comprend une matière bio-absorbable.

55. Appareil selon la revendication 51, comportant en outre un raccord sur l'extrémité proximale du corps pour relier de façon séparable le corps à un dispositif de distribution.

56. Appareil selon la revendication 49 ou la revendication 51, comportant en outre une tige allongée s'étendant depuis l'extrémité proximale du corps.
